# EUROPEAN PATENT SPECIFICATION

(11) **EP 2 857 500 B1**
(45) Date of publication and mention of the grant of the patent: **31.01.2018**
(21) Application number: 13793805.6
(22) Date of filing: 24.05.2013
(51) Int. Cl.: C12N 5/071, C12N 15/09

(54) **METHOD FOR PRODUCING PANCREATIC HORMONE-PRODUCING CELL, PANCREATIC HORMONE-PRODUCING CELL, AND DIFFERENTIATION/INDUCTION PROMOTER**
VERFAHREN ZUR HERSTELLUNG VON PANKREASHORMONPRODUZIERENDER ZELLE, PANKREASHORMONPRODUZIERENDE ZELLE UND DIFFERENZIERUNGS-/INDUKTIONSIONENPROMOTOR
PROCÉDÉ DE PRODUCTION D'UNE CELLULE PRODUISANT UNE HORMONE PANCRÉATIQUE, CELLULE DE PRODUCTION D'HORMONE PANCRÉATIQUE, ET PROMOTEUR DE DIFFÉRENTIATION/INDUCTION

(30) Priority: 25.05.2012 JP 2012120281
(43) Date of publication of application: 08.04.2015
(73) Proprietor: Saitama Medical University, Iruma-gun, Saitama 350-0495 (JP)
(72) Inventor: TOYOSHIMA, Hideo, Iruma-gun Saitama 350-0495 (JP); OKAZAKI, Yasushi, Iruma-gun Saitama 350-0495 (JP); YOKOO, Tomotaka, Iruma-gun Saitama 350-0495 (JP); SUGAHARA, Izumi, Iruma-gun Saitama 350-0495 (JP)
(74) Representative: Cabinet Plasseraud
(86) International application number: PCT/JP2013/064469
(87) International publication number: WO 2013/176249

(56) References cited:
- WO-A1-2009/013794
- WO-A1-2009/013794
- WO-A1-2009/048675
- WO-A2-2007/103282
- JP-A- 2007 209 214
- JIANG WEI ET AL: "In vitro derivation of functional insulin-producing cells from human embryonic stem cells", CELL RESEARCH - XIBAO YANJIU, NATURE PUBLISHING GROUP, GB, CN, vol. 17, no. 4, 10 April 2007 (2007-04-10) , pages 333-344, XP002455184, ISSN: 1001-0602, DOI: 10.1038/CR.2007.28
- YUYA KUNISADA ET AL: "Small molecules induce efficient differentiation into insulin-producing cells from human induced pluripotent stem cells", STEM CELL RESEARCH, vol. 8, no. 2, 1 March 2012 (2012-03-01), pages 274-284, XP055094959, ISSN: 1873-5061, DOI: 10.1016/j.scr.2011.10.002
- KEVIN A D'AMOUR ET AL: "Production of pancreatic hormone-expressing endocrine cells from human embryonic stem cells", NATURE BIOTECHNOLOGY, NATURE PUBLISHING GROUP, US, vol. 24, no. 11, 1 November 2006 (2006-11-01), pages 1392-1400, XP002650232, ISSN: 1087-0156, DOI: 10.1038/NBT1259 [retrieved on 2006-10-19]
- D'AMOUR, K.A. ET AL.: 'Production of pancreatic hormone-expressing endocrine cells from human embryonic stem cells' NATURE BIOTECHNOLOGY vol. 24, 2006, pages 1392 - 1401, XP002650232
- KUNISADA, Y. ET AL.: 'Small molecules induce efficient differentiation into insulin- producing cells from human induced pluripotent stem cells' STEM CELL RESEARCH vol. 8, no. 2, March 2012, pages 274 - 284, XP055094959
- JIANG, W. ET AL.: 'In vitro derivation of functional insulin-producing cells from human embryonic stem cells' CELL RESEARCH vol. 17, 2007, pages 333 - 344, XP002455184

## Description

### TECHNICAL FIELD

The present document describes a method of producing a pancreatic hormone-producing cell from a pluripotent stem cell such as an induced pluripotent stem cell (hereinafter also referred to as a "iPS cell") and an embryonic stem cell (hereinafter also referred to as an "ES cell") or a pancreas tissue stem/precursor cell, and also discloses a pancreatic hormone-producing cell thus produced. The present document further describes a differentiation-inducing promoter used for the production method thereof.

### BACKGROUND ART

Pancreas is an organ which comprises endocrine cells and exocrine cells, and plays an important role in both internal secretion and external secretion. Endocrine cells play a role in producing/secreting a pancreatic hormone. It is known that glucagon, insulin, somatostatin and pancreatic polypeptides are secreted from α cells, β cells, δ cells and PP cells, respectively. In particular, insulin shows an effect for reducing a blood glucose level, and plays an important role in maintaining blood glucose at the right concentration.

In recent years, a large number of methods for inducing differentiation of a pluripotent stem cell or a pancreas tissue stem/precursor cell into a pancreatic hormone-producing cell have been reported (see Nonpatent Literatures 1 to 4, Patent Literatures 1 to 6 and so on). If a pancreatic hormone-producing cell can be efficiently obtained by such differentiation-inducing methods, an alternative method of treating diabetes may become possible, substituting for pancreatic islet transplantation. Further, it is thought that rejection problems may also be eliminated since a pancreatic hormone-producing cell can be obtained from a pluripotent stem cell or a pancreas tissue stem/precursor cell of a patient himself/herself.

However, no differentiation-inducing methods reported to date have shown sufficient differentiation-inducing efficiency into a pancreatic hormone-producing cell. For example, the differentiation-inducing efficiency into an insulin-producing cell reported in Nonpatent Literature 1 is about 12%. Therefore, an efficient differentiation-inducing method is desired which is capable of inducing differentiation of a pluripotent stem cell or a pancreas tissue stem/precursor cell into a pancreatic hormone-producing cell. Further, in Nonpatent Literature 3, the pdx1 gene is introduced into a mouse ES cell, and cultured to induce differentiation into an insulin-producing cell, but a differentiation-inducing method without using a gene introduction is preferred in view of safety.

Patent Document 1: PCT International Application, Publication No. 2007/103282
Patent Document 2: PCT International Application, Publication No. 2005/063971
Patent Document 3: PCT International Application, Publication No. 2009/048675
Patent Document 4: PCT International Application, Publication No. 2007/051038
Patent Document 5: PCT International Application, Publication No. 2006/108361
Patent Document 6: PCT International Application, Publication No. 2008/066199
Non-Patent Document 1: D'Amour,K.A. et al., Nature Biotechnology, 24, pp.1392-1401(2006)
Non-Patent Document 2: Wei Jiang et al., Cell Research, 17, pp.333-344(2007)
Non-Patent Document 3: Miyazaki,S. et al., Diabetes, 53, pp.1030-1037(2004)
Non-Patent Document 4: Yuya Kunisada et al.,Stem Cell Research, 8, pp.274-284(2012)

### DISCLOSURE OF THE INVENTION

### Problems to be Solved by the Invention

The present invention is made in view of such previous actual situations. Described herein are a method for producing (a method for inducing differentiation into) a pancreatic hormone-producing cell in which differentiation of a pluripotent stem cell or a pancreas tissue stem/precursor cell into the pancreatic hormone-producing cell can be efficiently induced, and the pancreatic hormone-producing cell thus produced Also described is a differentiation-inducing promoter used for the production method.

### Means for Solving the Problems

The present inventors conducted extensive studies in order to solve the above problems. As a result, the present inventors found that differentiation of a pluripotent stem cell or a pancreas tissue stem/precursor cell into a pancreatic hormone-producing cell can be efficiently induced by adding a polypeptide known as human TM4SF20 or a culture supernatant of cells into which DNA encoding human TM4SF20 is incorporated as a foreign gene to a culture medium. The present invention is completed based on the above findings, and more specifically provides the follows.

A method of producing a pancreatic hormone-producing cell from a pluripotent stem cell or a pancreas tissue stem/precursor cell, the method comprising the steps of:
(A1) culturing the pluripotent stem cell in the presence of a growth factor belonging to the TGF-β (transforming growth factor β) superfamily,
(B1) culturing the cell obtained in the step (A1) in the presence of FGF (fibroblast growth factor),
(C1) culturing the cell obtained in the step (B1) in the presence of retinoid,
(D1) culturing the cell obtained in the step (C1) in the presence of a γ-secretase inhibitor and
(E1) culturing the cell obtained in the step (D1) in the presence of at least one factor selected from the group consisting of exendin-4, HGF (hepatocyte growth factor), IGF-1 (insulin like growth factor-1) and nicotinamide,
wherein at least one of the steps (A1) to (E1) is a differentiation-inducing process from the pluripotent stem cells or the pancreas tissue stem/precursor cell to the pancreatic hormone-producing cell, and at least one differentiation-inducing promoter selected from (1) to (3) is added to a culture medium in at least one of the steps (A1) to (E1) :
(1) a polypeptide consisting of an amino acid sequence encoded by DNA consisting of the base sequence set forth in SEQ ID NO. 1,
(2) a polypeptide consisting of an amino acid sequence having one or several amino acid substitutions, deletions, and/or additions in the amino acid sequence encoded by DNA consisting of the base sequence set forth in SEQ ID NO. 1, and having a differentiation-inducing promotion effect into a pancreatic hormone-producing cell,
(3) a culture supernatant of cells into which DNA consisting of the base sequence set forth in SEQ ID NO. 1 or DNA hybridizing with DNA consisting of a base sequence complementary to the DNA consisting of the base sequence set forth in SEQ ID NO: 1 under stringent conditions is incorporated as a foreign gene, wherein the DNA hybridizing with DNA consisting of the base sequence complementary to the DNA consisting of the base sequence set forth in SEQ ID NO: 1 encodes a polypeptide having a differentiation-inducing promotion effect into a pancreatic hormone-producing cell.

A method of producing a pancreatic hormone-producing cell from a pluripotent stem cell or a pancreas tissue stem/precursor cell, the method comprising the steps of:
(A2) culturing the pluripotent stem cell in the presence of at least one factor selected from the group consisting of a growth factor belonging to the TGF-β super family, a growth factor belonging to Wnt (wingless MMTV integration site) family and a GSK-3 (glycogen synthase kinase 3) inhibitor,
(B2) culturing the cell obtained in the step (A2) in the presence of a growth factor belonging to the TGF-β superfamily,
(C2) culturing the cell obtained in the step (B2) in the presence of retinoid,
(D2) culturing the cell obtained in the step (C2) in the presence of at least one factor selected from the group consisting of a cAMP (cyclic adenosine monophosphate) increasing agent, dexamethasone, a TGF-β1 type acceptor inhibitor and nicotinamide,
wherein at least one of the steps (A2) to (D2) is a differentiation-inducing process from the pluripotent stem cell or the pancreas tissue stem/precursor cell to the pancreatic hormone-producing cell, and
at least one differentiation-inducing promoter selected from (1) to (3) as defined above is added to a culture medium in at least one of the steps (A2) to (D2).

A method of producing a pancreatic hormone-producing cell from a pluripotent stem cell or a pancreas tissue stem/precursor cell, the method comprising the steps of:
(A3) culturing the pancreas tissue stem/precursor cell in the absence of a growth factor belonging to the TGF-β superfamily, retinoid, FGF and nicotinamide,
(B3) culturing the cell obtained in the step (A3) in the presence of a growth factor belonging to the TGF-β superfamily,
(C3) culturing the cell obtained in the step (B3) in the presence of retinoid,
(D3) culturing the cell obtained in the step (C3) in the presence of FGF and
(E3) culturing the cell obtained in the step (D3) in the presence of nicotinamide,
wherein at least one of the steps (A3) to (E3) is a differentiation-inducing process from the pluripotent stem cell or the pancreas tissue stem/precursor cell to the pancreatic hormone-producing cell, and
at least one differentiation-inducing promoter selected from (1) to (3) as defined above is added to a culture medium in at least one of the steps (A3) to (E3).

Is also described herein a pancreatic hormone-producing cell artificially produced by the method described above.

Use of a differentiation-inducing promoter for inducing differentiation of a pluripotent stem cell or a pancreas tissue stem/precursor cell into a pancreatic hormone-producing cell, comprising at least one of:
(1) a polypeptide consisting of an amino acid sequence encoded by DNA consisting of the base sequence set forth in SEQ ID NO. 1,
(2) a polypeptide consisting of an amino acid sequence having one or several amino acid substitutions, deletions, and/or additions in the amino acid sequence encoded by DNA consisting of the base sequence set forth in SEQ ID NO. 1, and having a differentiation-inducing promotion effect into a pancreatic hormone-producing cell,
(3) a culture supernatant of cells into which DNA consisting of the base sequence set forth in SEQ ID NO. 1 or DNA hybridizing with DNA consisting of a base sequence complementary to the DNA consisting of the base sequence set forth in SEQ ID NO: 1 under stringent conditions is incorporated as a foreign gene, wherein the DNA hybridizing with DNA consisting of the base sequence complementary to the DNA consisting of the base sequence set forth in SEQ ID NO: 1 encodes a polypeptide having a differentiation-inducing promotion effect into a pancreatic hormone-producing cell.

### Effects of the Invention

Are described herein a method for (a method of inducing differentiation into) a pancreatic hormone-producing cell in which differentiation of a pluripotent stem cell or a pancreas tissue stem/precursor cell into the pancreatic hormone-producing cell can be efficiently induced, and the pancreatic hormone-producing cell thus produced. The present document also describes a differentiation-inducing promoter used for the production method thereof.

### BRIEF DESCRIPTION OF THE DRAWINGS

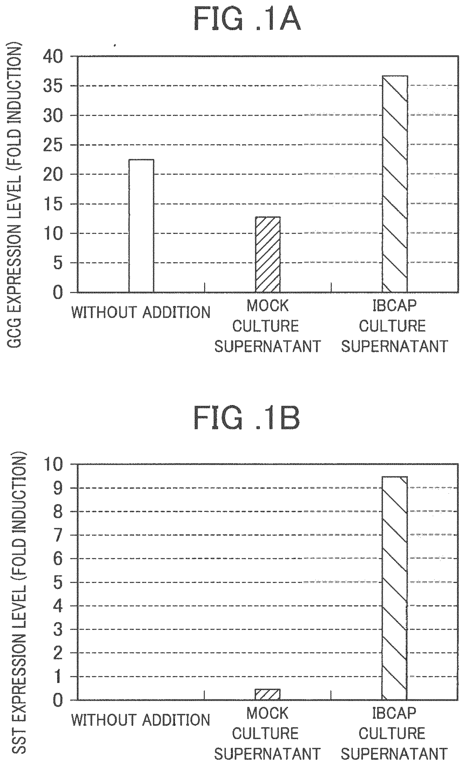

Fig. 1 shows the expression levels of glucagon (GCG) and somatostatin (SST) in a case where a culture supernatant of cells which are transfected with an expression vector for a polypeptide encoded by DNA consisting of the base sequence set forth in SEQ ID NO. 1 (IBCAP) is added in the final stage (the steps (D1), (E1)) of a differentiation-inducing process from a human iPS cell into a pancreatic hormone-producing cell.

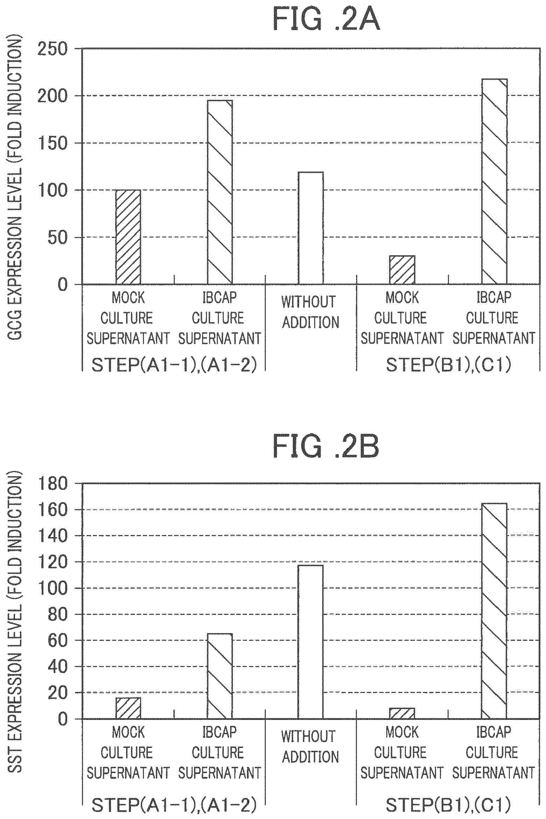

Fig. 2 shows the expression levels of glucagon (GCG) and somatostatin (SST) in a case where a culture supernatant of cells which are transfected with an expression vector for a polypeptide encoded by DNA consisting of the base sequence set forth in SEQ ID NO. 1 (IBCAP) is added in the initial stage (the steps (A1-1), (A1-1)) or the intermediate stage (the steps (B1), (C1)) of a differentiation-inducing process from a human iPS cell into a pancreatic hormone-producing cell.

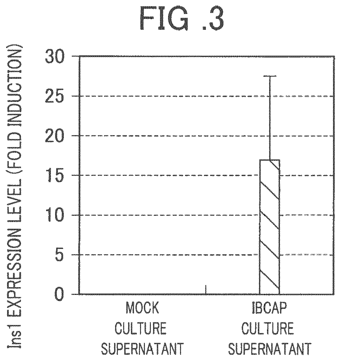

Fig. 3 shows the expression level of mouse insulin-1 (Ins1) in a case where a culture supernatant of cells which are transfected with an expression vector for a polypeptide encoded by DNA consisting of the base sequence set forth in SEQ ID NO. 1 (IBCAP) is added in the final stage (the step (E3)) of a differentiation-inducing process from a mouse pancreas tissue stem/precursor cell into a pancreatic hormone-producing cell.

### PREFERRED MODE FOR CARRYING OUT THE INVENTION

The method of producing a pancreatic hormone-producing cell described herein is characterized in that a specific differentiation-inducing promoter is added to a culture medium during a differentiation-inducing process from a pluripotent stem cell or a pancreas tissue stem/precursor cell into a pancreatic hormone-producing cell. In the followings, a pluripotent stem cell, a pancreas tissue stem/precursor cell and a differentiation-inducing promoter are first described in that order, and then a specific method of producing (a method of inducing differentiation into) a pancreatic hormone-producing cell is described.

### Pluripotent stem cell

A pluripotent stem cell means a self-replication competent stem cell having a differentiation capability (pluripotency) into a differentiated cell belonging to at least each of three germ layers (ectoderm, mesoderm, endoderm). For example, induced pluripotent stem cells (iPS cells), embryonic stem cells (ES cells), embryonic cancer cells (EC cells), adult pluripotent stem cells (APS cells) and the like are encompassed by this term. In the production method described herein, among others, an induced pluripotent stem cell (iPS cell) or an embryonic stem cell (ES cell) is preferably used.

An iPS cell refers to a cell having pluripotency obtained by reprogramming a somatic cell. Multiple groups have successfully produced iPS cells, including the group of Dr. Yamanaka and colleagues at Kyoto University, the group of Dr. Jaenisch and colleagues at Massachusetts Institute of Technology, the group of Dr. Thomson and colleagues at University of Wisconsin, the group of Dr. Hochedlinger and colleagues at Harvard University and so on.

There is no particular limitation for the somatic cell used for producing an iPS cell, and any somatic cells can be used. That is, any cells among those constituting the living organism can be used except for germ cells, and either differentiated somatic cells or undifferentiated stem cells may be used. There is no particular limitation for the origin of a somatic cell, and it can be any of mammal, bird, fish, reptile, amphibian, but mammal is preferred, and human or mouse is preferred among others. When a human somatic cell is used, a somatic cell from any of a fetus, a newborn infant and an adult may be used.

In order to produce an iPS cell from a somatic cell, at least one reprogramming gene needs to be introduced into the somatic cell for reprogramming. A reprogramming gene refers to one encoding a reprogramming factor having a function to reprogram a somatic cell into an iPS cell. Combinations of the reprogramming genes for producing a human iPS cell can include, for example, (i) to (iv) shown below, but not limited to these examples.
(i) the OCT gene, the KLF gene, the SOX gene, the MYC gene
(ii) the OCT gene, the SOX gene, the NANOG gene, the LIN28 gene
(iii) the OCT gene, the KLF gene, the SOX gene, the MYC gene, the hTERT gene, the SV40 large T gene
(iv) the OCT gene, the KLF gene, the SOX gene

Meanwhile, an ES cell refers to a stem cell having pluripotency and self-replication capability There is no particular limitation for the origin of an ES cell, but mammal is preferred, and human or mouse is preferred among others. As an ES cell, for example, a cell into which a reporter gene is introduced near the PDX1 gene can be used so that the degree of differentiation thereof can be easily determined.

### Pancreas tissue stem/precursor cell

A pancreas tissue stem/precursor cell refers to a tissue stem/precursor cell having pluripotency and self-replication capability which are present in the pancreas of an animal. There is no particular limitation for the origin of a pancreas tissue stem/precursor cell, but mammal is preferred, and human or mouse is preferred among others. There is no particular limitation for the method of separating a tissue stem/precursor cell from pancreas, and any publicly known methods can be used. For example, in the fetal pancreas, PDX1 is known as a marker molecule for a pancreas tissue stem/precursor cell (Jonsson, J. et al., Nature, 371, pp. 606-609 (1994); Offield, M. F. et al., Development, 22, pp. 983-995 (1996)). A fetus PDX1 expressing cell differentiates into an endocrine cell, an exocrine cell and a pancreatic duct cell, and produces all types of cells present in the adult pancreas. Accordingly, a pancreas tissue stem/precursor cell may be separated using PDX1 as a marker molecule.

Note that the above pancreas tissue stem/precursor cell may be established, or may not be established.

### Differentiation-inducing promoter

The differentiation-inducing promoter used in the method of producing a pancreatic hormone-producing cell described herein is at least one selected from (1) to (3) below.
(1) a polypeptide consisting of an amino acid sequence encoded by DNA consisting of the base sequence set forth in SEQ ID NO. 1,
(2) a polypeptide consisting of an amino acid sequence having one or several amino acid substitutions, deletions, and/or additions in the amino acid sequence encoded by DNA consisting of the base sequence set forth in SEQ ID NO. 1, and having a differentiation-inducing promotion effect into a pancreatic hormone-producing cell,
(3) a culture supernatant of cells into which DNA consisting of the base sequence set forth in SEQ ID NO. 1 or DNA hybridizing with DNA consisting of a base sequence complementary to the DNA consisting of the base sequence set forth in SEQ ID NO: 1 under stringent conditions is incorporated as a foreign gene, wherein the DNA hybridizing with DNA consisting of the base sequence complementary to the DNA consisting of the base sequence set forth in SEQ ID NO: 1 encodes a polypeptide having a differentiation-inducing promotion effect into a pancreatic hormone-producing cell.

DNA consisting of the base sequence set forth in SEQ ID NO. 1 is a full length of 2308 bp known as DNA encoding human TM4SF20 (NCBI: LOCUS NM 024795). CDS of this DNA is 38..727, and encodes a polypeptide having a sequence of 229 amino acids set forth in SEQ ID NO. 3 (see SEQ ID NO. 2). In the production method described herein, this polypeptide can be used as a differentiation-inducing promoter.

Further, in the method of producing a pancreatic hormone-producing cell described herein, a polypeptide consisting of an amino acid sequence having one or several amino acid substitutions, deletions, and/or additions in the amino acid sequence of the aforementioned polypeptide (hereinafter referred to as a "modified polypeptide") may also be used as a differentiation-inducing promoter as long as the differentiation-inducing promotion effect into a pancreatic hormone-producing cell is maintained. It is already known that a polypeptide consisting of an amino acid sequence modified by substituting, deleting, and/or adding one or several amino acids to a certain amino acid sequence can maintain its biological activity (see Mark, D. F. et al., Proc. Natl. Acad. Sci. USA, 81, pp. 5662-5666 (1984); Zoller, M. J. et al., Nucleic Acids Research, 10, pp. 6487-6500 (1982); Wang, A. et al., Science, 224, pp. 1431-1433; Dalbadie-McFarland, G. et al., Proc. Natl. Acad. Sci. USA, 79, pp. 6409-6413 (1982); and so on).

In this case, when substituting one or several amino acids with other amino acids in the amino acid sequence of the aforementioned polypeptide, the properties of an amino acid side chain before and after substitution is preferably conserved. Properties of an amino acid side chain include hydrophobic amino acids (A, I, L, M, F, P, W, Y, V), hydrophilic amino acids (R, D, N, C, E, Q, G, H, K, S, T), amino acids having an aliphatic side chain (G, A, V, L, I, P), amino acids having a hydroxyl group-containing side chain (S, T, Y), amino acids having a sulfur atom-containing side chain (C, M), amino acids having a carboxylic acid and amide-containing side chain (D, N, E, Q), amino acids having a base-containing side chain (R, K, H), amino acids having an aromatic-containing side chain (H, F, Y, W) (all alphabets in parenthesis represent the one letter notation for amino acids).

When substituting, deleting and/or adding one or several amino acids, the number thereof may be 1 to 20, or may be 1 to 15, or may be 1 to 10, or may be 1 to 5.

Further, the homology between a modified polypeptide and the original polypeptide is preferably 80% or more, more preferably 90% or more, even more preferably 93% or more, in particular preferably 95% or more and most preferably 98% or more.

Note that amino acids at positions 1 to 163 and 179 to 229 in SEQ ID NO. 3 are regions highly conserved among different biological species. Therefore, amino acids in these regions are preferably conserved before and after modification.

The aforementioned polypeptide and modified polypeptide may be chemically synthesized, or may be obtained by genetic engineering. For example, DNA consisting of the base sequence set forth in SEQ ID NO. 1 or DNA hybridizing with DNA consisting of a base sequence complementary with the aforementioned DNA under stringent conditions is incorporated into a cultivable host cell as a foreign gene, and the host cell is cultured to express the gene.

Then the aforementioned polypeptide and modified polypeptide can be obtained from a culture supernatant thereof. As a host cell, known cells such as bacteria, yeast, insect cells and animal cells may be appropriately used. Animal cells include the HEK293 cell, the HEK293T cell, the CHO-K1 cell, the COS cell and the like.

The term "DNA hybridized under stringent conditions" as used herein means DNA obtainable by utilizing the colony hybridization method, the plaque hybridization method, the southern blotting hybridization method and the like using a specific DNA (DNA consisting of a base sequence complementary with DNA consisting of the base sequence set forth in SEQ ID NO. 1) as a probe. For example, they include DNA identifiable by performing hybridization at 65°C in the presence of 0.7 to 1.0 M sodium chloride using a filter in which DNA from a colony and plaque is immobilized, and then washing the filter under a temperature condition of 65°C with a 0.1 to 2 x SSC solution (the composition of 1 x SSC: 150 mM sodium chloride, 15 mM sodium citrate) (if desired, see Molecular Cloning: A Laboratory Manual, 2nd Ed., Cold Spring Harbor Laboratory, Cold Spring Harbor, NY., 1989., and the like). The homology with a base sequence of DNA used as a probe for a base sequence of DNA to be hybridized under stringent conditions is preferably 80% or more, more preferably 90% or more, even more preferably 93% or more, in particular preferably 95% or more and most preferably 98% or more.

Separation/purification of these polypeptides and modified polypeptides can be performed by methods commonly used in peptide chemistry such as an ion exchange resin, the partition chromatography, the gel chromatography, the reversed phase chromatography, for example.

Further, in the method of producing a pancreatic hormone-producing cell described herein, a culture supernatant containing the aforementioned polypeptide and modified polypeptide can also be used as a differentiation-inducing promoter. When using a culture supernatant as a differentiation-inducing promoter, the culture supernatant is preferably concentrated by ultrafiltration and the like. Further, unwanted chemicals and the like may be removed by dialysis, if desired.

### Method for inducing differentiation of pluripotent stem cell into pancreatic hormone-producing cell (method of producing pancreatic hormone-producing cell)

In the method of producing a pancreatic hormone-producing cell described herein, the aforementioned differentiation-inducing promoter is added to a culture medium in a differentiation-inducing process from a pluripotent stem cell into a pancreatic hormone-producing cell. There is no particular limitation for the method of inducing differentiation of a pluripotent stem cell into a pancreatic hormone-producing cell, and any publicly known methods can be used. When adding a polypeptide or a modified polypeptide as a differentiation-inducing promoter, the concentration thereof is preferably 10 to 200 ng/mL, more preferably 50 to 180 ng/mL and even more preferably 60 to 150 ng/mL. Further, when adding a culture supernatant as a differentiation-inducing promoter, the concentration thereof is preferably 0.5 to 20% (v/v), more preferably 1 to 10% (v/v) and even more preferably 1.5 to 5% (v/v) .

Below, two methods will be described as examples of a method for inducing differentiation of a pluripotent stem cell into a pancreatic hormone-producing cell (a method of producing a pancreatic hormone-producing cell). However, the method of producing a pancreatic hormone-producing cell described herein is not limited to these examples.

### [First differentiation-inducing method]

A first differentiation-inducing method is performed in accordance with the method described in Nonpatent Literature 1.

The first differentiation-inducing method comprises the steps (A1) to (E1) as shown below. In at least one of these steps, the aforementioned differentiation-inducing promoter is added to a culture medium. Addition of a differentiation-inducing promoter is preferably performed in at least one of the steps (A1) to (C1), more preferably in at least one of the steps (B1) to (C1). Note that when adding a differentiation-inducing promoter in a certain step, it may be added at the beginning of the step or may be added in the middle of the step.
(A1) The step of culturing a pluripotent stem cell in the presence of a growth factor belonging to the TGF-β superfamily.
(B1) The step of culturing the cell obtained in the step (A1) in the presence of FGF.
(C1) The step of culturing the cell obtained in the step (B1) in the presence of retinoid.
(D1) The step of culturing the cell obtained in the step (C1) in the presence of a γ-secretase inhibitor.
(E1) The step of culturing the cell obtained in the step (D1) in the presence of at least one factor selected from the group consisting of exendin-4, HGF, IGF-1 and nicotinamide.

### (Step (A1))

In the step (A1), a pluripotent stem cell is cultured in the presence of a growth factor belonging to the TGF-β superfamily.

Growth factors belonging to the TGF-β superfamily include activin, nodal, BMP (an osteogenesis protein) and the like, and among these, activin is preferred. These growth factors belonging to the TGF-β superfamily are known to promote differentiation of a pluripotent stem cell into an embryonic endoderm cell (see Nonpatent Literature 1, Patent Literatures 1 to 3 and so on). Examples of activin include activin A, activin B, activin AB and the like, and among these, activin A is preferred.

The concentration of a growth factor belonging to the TGF-β superfamily is preferably 5 to 250 ng/mL, more preferably 10 to 200 ng/mL and even more preferably 50 to 150 ng/mL.

Further, a growth factor belonging to the Wnt family is preferably added to a culture medium in the step (A1). By adding a growth factor belonging to the Wnt family along with a growth factor belonging to the TGF-β superfamily, differentiation efficiency into an embryonic endoderm cell can be enhanced.

Growth factors belonging to the Wnt family include Wnt1, Wnt3a, Wnt5a, Wnt7a and the like, and Wnt1, Wnt3a are preferred, and Wnt3a is more preferred.

The concentration of a growth factor belonging to the Wnt family is preferably 1 to 1000 ng/mL, more preferably 10 to 100 ng/mL and even more preferably 10 to 50 ng/mL.

Note that in the step (A1), a GSK-3 inhibitor (for example, CHIR) may be added instead of a growth factor belonging to the Wnt family. A GSK-3 inhibitor (for example, CHIR) is known to activate the Wnt signaling pathway (J. Biol. Chem. 277 (34), pp. 30998-31004 (2002)).

Note that in the step (A1), an additional factor which can possibly enhance differentiation efficiency into an embryonic endoderm cell may be added to a culture medium. Additional factors include, for example, PDGF (platelet derived growth factor), EGF (epidermal growth factor), VEGF (vascular endothelial cell growth factor), KGF (keratinocyte growth factor), HGF, NGF (nerve growth factor), GDF (growth differentiation factor), GLP (glucagon-like peptide), nicotinamide, exendin 4, retinoic acid, ethanolamine, parathyroid hormone, progesterone, aprotinin, hydrocortisone, gastrin, steroid alkaloid, copper chelators (triethylene pentamine and the like), forskolin, sodium butyrate, noggin, valproic acid, tricostatin A, Indian hedgehog, sonic hedgehog, proteasome inhibitors, notch pathway inhibitors, hedgehog pathway inhibitors and the like.

For a container used for culture, a culture plate is preferred which is coated with a scaffold of a biocompatible material in view of differentiation-inducing ability, functional expression ability, viability and the like. Scaffolds include laminin, fibronectin, collagen, heparan sulphate proteoglycan, gelatin, entactin, polyornithine and the like. As commercially available products, MATRIGEL™, growth factor reduced MATRIGEL™ from Becton Dickinson and the like are available. In particular, a culture plate coated with MATRIGEL™ is preferably used.

A culture medium used for culture is prepared by adding various nutrient sources and other components required for maintaining cell growth to a basal medium which can be used for culturing animal cells.

Basal media include a RPMI1640 culture medium, a DMEM culture medium, a CMRL1066 culture medium, Ham's F12 culture medium, an Eagle MEM culture medium, a Glasgow MEM culture medium, an IMEM Zinc Option culture medium, an IMDM culture medium, a William E culture medium, a Fischer culture medium, a McCoy culture medium, a BME culture medium, an αMEM culture medium, a BGJb culture medium, a Medium 199 culture medium, or mixed media thereof and the like.

Nutrient sources include carbon sources such as glycerol, glucose, fructose, sucrose, lactose, starch, dextrin; hydrocarbons such as fatty acid, fat and oil, lecithin, alcohol; nitrogen sources such as ammonium sulfate, ammonium nitrate, ammonium chloride, urea, sodium nitrate: inorganic salts such as sodium salts, potassium salts, magnesium salts, calcium salts, phosphates; various vitamins; various amino acids; and the like.

Other components include antibiotics such as penicillin, streptomycin; cholera toxin; insulin; transferrin; selenious acid; albumin; 2-mercaptoethanol; serum or serum substitutes; and the like. With regards to insulin, transferrin and selenious acid, ITS-X, ITS-A, ITS-G from Invitrogen and the like are available as commercial products. Further, as serum substitutes, a B-27™ supplement, a N-2 supplement, a Knockout™ serum substitute from Invitrogen and the like are available as commercial products.

In this case, in order to enhance the differentiation efficiency in the step (A1), the sufficiently low content of insulin, IGF and the like in a culture medium is known to be important (see WO2006/020919). For this reason, a serum free culture medium or a low serum culture medium is preferably used in the step (A1) (see Nonpatent Literature 1, Patent Literatures 1 to 3). The concentration of serum is preferably 0 to 2% (v/v), more preferably 0 to 1% (v/v) and even more preferably 0 to 0.5% (v/v).

According to a preferred embodiment, a serum free or low serum RPMI1640 culture medium is used which is supplemented with activin A, Wnt3a, antibiotics such as penicillin and streptomycin, L-glutamine or a dipeptide comprising L-glutamine.

The culturing period in the step (A1) is, for example, 1 to 6 days, preferably 2 to 4 days.

The progress of differentiation into an embryonic endoderm cell can be evaluated by morphological observation or alternatively by observing gene expression using RT-PCR. As differentiation of a pluripotent stem cell into an embryonic endoderm cell progresses, expressions of marker genes for a stem cell OCT4, NANOG, SOX2, ECAD and the like are decreased while expressions of marker genes for an embryonic endoderm cell SOX17, CER, FOXA2, CXCR4 and the like are promoted.

Note that a reduced concentration of serum in a culture medium is required in order to enhance differentiation efficiency into an embryonic endoderm cell while a higher concentration of serum in the culture medium is preferred in order to increase cell viability.

Therefore, the step (A1) is preferably divided into a step (A1-1) of culturing in a first culture medium without serum and a step (A1-2) of culturing in a second culture medium with low serum.

The first culture medium used in the step (A1-1) may be the same as that described above except that it is serum free. That is, the first culture medium may contain a growth factor belonging to the TGF-β superfamily, and in addition may contain a growth factor belonging to the Wnt family. The first culture medium preferably contains a growth factor belonging to the Wnt family.

The culturing period in the step (A1-1) is, for example, 1 to 3 days, more preferably 1 to 2 days. By this culturing, differentiation of a pluripotent stem cell into a mesendoderm cell will progress.

Progress of differentiation inducing into a mesendoderm cell may be evaluated by morphological observation or alternatively by observing gene expression using RT-PCR. As differentiation of a pluripotent stem cell into a mesendoderm cell progresses, expressions of marker genes for a stem cell OCT4, NANOG, SOX2, ECAD and the like are decreased while expressions of marker genes for a mesendoderm cell BRA, FGF4, WNT3, NCAD and the like are promoted.

The second culture medium used in the step (A1-2) may be the same as that described above except that it is of low serum. That is, the second culture medium may contain a growth factor belonging to the TGF-β superfamily, and in addition may contain a growth factor belonging to the Wnt family. The concentration of serum is preferably 0.05 to 2% (v/v), more preferably 0.05 to 1% (v/v) and even more preferably 0.1 to 0.5% (v/v).

The culturing period in the step (A1-2) is, for example, 1 to 3 days, more preferably 1 to 2 days. By this culturing, differentiation of a mesendoderm cell into an embryonic endoderm cell will progress.

As described above, progress of differentiation inducing into an embryonic endoderm may be evaluated by morphological observation or alternatively by observing gene expression using RT-PCR.

Note that before the next step (B1), the cell obtained may be concentrated, isolated and/or purified by known methods.

### (Step (B1))

In the step (B1), the cell obtained in the step (A1) is cultured in the presence of FGF.

Examples of FGF include FGF-1, FGF-2 (bFGF), FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, FGF-23 and the like, and FGF-2 (bFGF), FGF-5, FGF-7, FGF-10 are preferred.

The concentration of FGF is preferably 5 to 150 ng/mL, more preferably 10 to 100 ng/mL and even more preferably 20 to 80 ng/mL.

Further, in the step (B1), a hedgehog pathway inhibitor is preferably added to a culture medium. Differentiation efficiency can be enhanced by adding a hedgehog pathway inhibitor along with FGF.

Hedgehog pathway inhibitors include KAAD-cyclopamine (28-[2-[[6-[(3-phenylpropanoly)amino]hexanoly]amino]ethyl]-17β,23β-epoxyveratraman-3-one), analogs of KAAD-cyclopamine, jervine (17,23β-epoxy-3β-hydroxyveratraman-11-one), analogs of jervine, hedgehog pathway blocking antibodies and the like, and among these, KAAD-cyclopamine is preferred.

The concentration of a hedgehog pathway inhibitor is preferably 0.01 to 5 µM, more preferably 0.02 to 2 µM and even more preferably 0.1 to 0.5 µM.

A container used for culture may be the same as that used in the step (A1). A culture medium may be the same as that in the step (A1) except for each of the aforementioned factors and the concentration of serum in a culture medium. The concentration of serum in a culture medium is preferably 0.1 to 5% (v/v), more preferably 0.5 to 5% (v/v) and even preferably 1 to 5% (v/v).

Note that in a case where a low serum culture medium is used in the step (A1), a culture medium having a higher concentration of serum than that in the step (A1) is preferably used in the step (B1).

In a preferred embodiment, a low serum RPMI1640 culture medium is used which is supplemented with FGF-10, KAAD-cyclopamine, antibiotics such as penicillin and streptomycin, L-glutamine or a dipeptide containing L-glutamine.

The culturing period in the step (B1) is, for example, 1 to 6 days, preferably 2 to 4 days.

Progress of differentiation-inducing can be evaluated by morphological observation or alternatively by observing gene expression using RT-PCR. As differentiation progresses, expressions of genes such as HNF1B, HNF4A and the like are promoted.

Note that before the next step (C1), the cell obtained may be concentrated, isolated and/or purified by known methods.

### (Step (C1))

In the step (C1), the cell obtained in the step (B1) is cultured in the presence of retinoid.

Examples of retinoid include retinol, retinal, retinoic acid and the like, and among these, retinoic acid is preferred.

The concentration of retinoid is preferably 0.2 to 10 µM, more preferably 0.4 to 8 µM and even more preferably 1 to 4 µM.

Further, in the step (C1), a hedgehog pathway inhibitor is preferably added to a culture medium. Differentiation efficiency can be enhanced by adding a hedgehog pathway inhibitor along with retinoid.

Hedgehog pathway inhibitors include KAAD-cyclopamine, analogs of KAAD-cyclopamine, jervine, analogs of jervine, hedgehog pathway blocking antibodies and the like, and among these, KAAD-cyclopamine is preferred.

The concentration of a hedgehog pathway inhibitor is preferably 0.01 to 5 µM, more preferably 0.02 to 2 µM and even more preferably 0.1 to 0.5 µM.

Further, in the step (C1), FGF is preferably added to a culture medium. Differentiation efficiency can be enhanced by adding FGF along with retinoid.

Examples of FGF include FGF-1, FGF-2 (bFGF), FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, FGF-23 and the like, and FGF-2 (bFGF), FGF-5, FGF-7, FGF-10 are preferred.

The concentration of FGF is preferably 0.5 to 50 ng/mL, more preferably 1 to 25 ng/mL and even more preferably 2 to 10 ng/mL.

Further, in the step (C1), a growth factor belonging to the TGF-β superfamily may be added in a culture medium.

The concentration of a growth factor belonging to the TGF-β superfamily is preferably 5 to 250 ng/mL, more preferably 10 to 200 ng/mL and even more preferably 20 to 150 ng/mL.

A container used for culture may be the same as that used in the step (B1). A culture medium may be basically the same as that used in the step (B1) except for each of the aforementioned factors. However, instead of serum, a serum substitute is preferably added to the culture medium. As commercial products of a serum substitute, a B-27™ supplement, a N-2 supplement, a Knockout™ serum substitute from Invitrogen and the like are available, and among these the B-27™ supplement is preferred.

The concentration of the B-27™ supplement is preferably 0.1 to 10% (v/v), more preferably 0.2 to 5% (v/v) and even more preferably 0.4 to 2.5% (v/v). Note that the B-27™ supplement, which is commercially available as a 50x stock solution, may be added to the culture medium such that it is diluted by 5 to 500 times to give a concentration of the B-27™ supplement of 0.1 to 10% (v/v).

In a preferred embodiment, a serum-free DMEM/Ham's F12 culture medium is used which is supplemented with retinoic acid, KAAD-cyclopamine, FGF-10, antibiotics such as penicillin and streptomycin, a B-27™ supplement and the like.

The culturing period in the step (C1) is, for example, 1 to 6 days, preferably 2 to 4 days. Progress of differentiation-inducing can be evaluated by morphological observation or alternatively by observing gene expression using RT-PCR. As differentiation progresses, expressions of genes such as PDX1, HNF6, HLXB9 are promoted.

Note that before the next step (D1), the cell obtained may be concentrated, isolated and/or purified by known methods.

### [Step (D1)]

In the step (D1), the cell obtained in the step (C1) is cultured in the presence of a γ-secretase inhibitor.

γ-secretase inhibitors include DAPT (N-[N-(3,5-difluorophenacetyl-L-alanyl)]-S-phenylglycine-tert-butylester), L-685458 ([1S-benzyl-4R-[1-(1S-carbamoyl-2-phenethylcarbamoyl)-1S-3-methylbutylcarbamoyl]-2R-hydroxy-5-phenethylpentyl]carbamic acid tert-butyl ester) and the like, and among these, DAPT is preferred.

The concentration of a γ-secretase inhibitor is preferably 1 to 50 µM, more preferably 2 to 40 µM and even more preferably 5 to 20 µM.

Further, in the step (D1), exendin-4 is preferably added to a culture medium. Differentiation efficiency can be enhanced by adding exendin-4 along with a γ-secretase inhibitor.

The concentration of exendin-4 is preferably 5 to 150 ng/mL, more preferably 10 to 100 ng/mL and even more preferably 20 to 80 ng/mL.

A container and a culture medium used for culture may be the same as those used in the step (C1). That is, a serum substitute is preferably added to a culture medium.

In a preferred embodiment, a serum-free DMEM/Ham's F12 culture medium is used which is supplemented with DAPT, exendin-4, antibiotics such as penicillin and streptomycin, a B-27™ supplement.

The culturing period in the step (D1) is, for example, 1 to 6 days, preferably 2 to 3 days.

Progress of differentiation-inducing can be evaluated by morphological observation or alternatively by observing gene expression using RT-PCR. As differentiation progresses, expressions of genes such as NKX6-1, NGN3, PAX4, NKX2-2 are promoted.

Note that before the next step (E1), the cell obtained may be concentrated, isolated and/or purified by known methods.

### (Step (E1))

In the step (E1), the cell obtained in the step (D1) is cultured in the presence of at least one factor selected from the group consisting of exendin-4, HGF, IGF-1 and nicotinamide.

With regards to exendin-4, HGF, IGF-1 and nicotinamide, two or more of them are preferably added, and more preferably three or more are added.

The concentration of exendin-4 is preferably 5 to 150 nM, more preferably 10 to 100 nM and even more preferably 20 to 80 nM.

The concentration of HGF is preferably 5 to 150 ng/mL, more preferably 10 to 100 ng/mL and even more preferably 20 to 80 ng/mL.

The concentration of IGF-1 is preferably 5 to 150 ng/mL, more preferably 10 to 100 ng/mL and even more preferably 20 to 80 ng/mL.

The concentration of nicotinamide is preferably 1 to 30 mM, more preferably 3 to 20 mM and even more preferably 5 to 15 mM.

A container and a culture medium used for culture may be the same as those used in the step (D1). That is, a serum substitute is preferably added to a culture medium.

In a preferred embodiment, a serum free CMRL1066 culture medium is used which is supplemented with exendin-4, HGF, IGF-1, antibiotics such as penicillin, streptomycin, a B-27™ supplement.

The culturing period in the step (E1) is, for example, 3 to 20 days, more preferably 3 to 10 days.

In this step (E1), a pancreatic hormone-producing cell can be obtained.

Progress of differentiation-inducing into a pancreatic hormone-producing cell can be evaluated by observing production of pancreatic hormones such as insulin, glucagon, somatostatin, or alternatively evaluated by observing gene expression using RT-PCR. As differentiation progresses, expression of at least one gene of INS, GCG, GHRL, SST, PPY and the like is promoted.

### Second differentiation-inducing method

A second differentiation-inducing method is performed in accordance with the method described in Nonpatent Literature 4.

The second differentiation-inducing method comprises the steps (A2) to (D2) described below. In at least one of these steps, the aforementioned differentiation-inducing promoter is added to a culture medium. The step of adding a differentiation-inducing promoter is preferably preformed in at least one of the steps (C2) to (D2), and in particular preferably performed in the step (D2). Note that when adding a differentiation-inducing promoter in a certain step, it may be added at the beginning of the step or may be added in the middle of the step.
(A2) The step of culturing a pluripotent stem cell in the presence of at least one factor selected from the group consisting of a growth factor belonging to the TGF-β superfamily, a growth factor belonging to the Wnt family and a GSK-3 inhibitor.
(B2) the step of culturing the cell obtained in the step (A2) in the presence of a growth factor belonging to the TGF-β superfamily.
(C2) the step of culturing the cell obtained in the step (B2) in the presence of retinoid.
(D2) The step of culturing the cell obtained in the step (C2) in the presence of at least one factor selected from the group consisting of a cAMP increasing agent, dexamethasone, a TGF-β1 type acceptor inhibitor and nicotinamide.

### (Step (A2))

In the step (A2), a pluripotent stem cell is cultured in the presence of at least one factor selected from the group consisting of a growth factor belonging to the TGF-β superfamily, a growth factor belonging to the Wnt family and a GSK-3 inhibitor.

Growth factors belonging to the TGF-β superfamily include activin, nodal, BMP and the like, and among these, activin is preferred. Examples of activin include activin A, activin B, activin AB and the like, and among these, activin A is preferred. The concentration of a growth factor belonging to the TGF-β superfamily is preferably 5 to 250 ng/mL, more preferably 10 to 200 ng/mL and even more preferably 50 to 150 ng/mL.

Growth factors belonging to the Wnt family include Wnt1, Wnt3a, Wnt5a, Wnt7a and the like, and Wnt1, Wnt3a are preferred, and Wnt3a is more preferred.

The concentration of a growth factor belonging to the Wnt family is preferably 1 to 1000 ng/mL, more preferably 10 to 100 ng/mL and even more preferably 10 to 50 ng/mL.

As a GSK-3 inhibitor, either a GSK-3α inhibitor or a GSK-3β inhibitor may be used, but a GSK-3β inhibitor is preferably used. Specific examples include CHIR99021 (6-[[2-[[4-(2,4-dichlorophenyl)-5-(5-methyl-1H-imidazole-2-yl)-2-pyrimidinyl]amino]ethyl]amino]-3-pyridinecarbonitrile), SB415286 (3-[(3-chloro-4-hydroxyphenyl)amino]-4-(2-nitrophenyl)-1H-pyrrole-2,5-dione), SB216763 (3-(2,4-dichlorophenyl)-4-(1-methyl-1H-indole-3-yl)-1H-pyrrole-2,5-dione), indirubin-3'-monooxime(3-[(3E)-3-(hydroxyimino)-2,3-dihydro-1H-indole-2-ylidene]-2,3-dihydro-1H-indole-2-one), kenpaullone (7,8-dihydro-9-bromoindolo[3,2-d][1]benzoazepin-6(5H)-one) and the like, and among these, CHIR99021 is preferred.

The concentration of a GSK-3 inhibitor is preferably 0.01 to 20 µM, more preferably 0.1 to 20 µM and even more preferably 1 to 5 µM.

Further, in the step (A2), an additional factor which can possibly enhance differentiation efficiency may be added to a culture medium. Additional factors include, for example, PDGF, EGF, VEGF, KGF, HGF, NGF, GDF, GLP, nicotinamide, exendin-4, retinoic acid, ethanolamine, parathyroid hormone, progesterone, aprotinin, hydrocortisone, gastrin, steroid alkaloid, copper chelators (triethylenepentamine and the like), forskolin, sodium butyrate, noggin, valproic acid, tricostatin A, Indian hedgehog, sonic hedgehog, proteasome inhibitors, notch pathway inhibitors, hedgehog pathway inhibitors and the like.

For a container used for culture, a culture plate coated with a scaffold of a biocompatible material is preferred in view of differentiation-inducing ability, functional expression ability, viability and the like. Scaffolds include laminin, fibronectin, collagen, heparan sulphate proteoglycan, gelatin, entactin, polyornithine and the like. As commercially available products, MATRIGEL™, growth factor reduced MATRIGEL™ from Becton Dickinson and the like are available. In particular, a culture plate coated with MATRIGEL™ is preferably used.

A culture medium used for culture is prepared by adding various nutrient sources and other components required for maintaining cell growth to a basal medium which can be used for culturing an animal cell.

Basal media include a RPMI1640 culture medium, a DMEM culture medium, a CMRL1066 culture medium, Ham's F12 culture medium, an Eagle MEM culture medium, a Glasgow MEM culture medium, an IMEM Zinc Option culture medium, an IMDM culture medium, a William E culture medium, a Fischer culture medium, a McCoy culture medium, a BME culture medium, an αMEM culture medium, a BGJb culture medium, a Medium 199 culture media, or mixed media thereof and the like.

Nutrient sources include carbon sources such as glycerol, glucose, fructose, sucrose, lactose, starch, dextrin; hydrocarbons such as fatty acid, fat and oil, lecithin, alcohol; nitrogen sources such as ammonium sulfate, ammonium nitrate, ammonium chloride, urea, sodium nitrate; inorganic salts such as sodium salts, potassium salts, magnesium salts, calcium salts, phosphates; various vitamins; various amino acids; and the like.

Other components include antibiotics such as penicillin, streptomycin; cholera toxin; insulin; transferrin; selenious acid; albumin; 2-mercaptoethanol; serum or serum substitutes; and the like. With regards to insulin, transferrin and selenious acid, ITS-X, ITS-A, ITS-G from Invitrogen and the like are available as commercial products. Further, as serum substitutes, a B-27™ supplement, a N-2 supplement, a Knockout™ serum substitute from Invitrogen and the like are available as commercial products.

In this case, in order to enhance differentiation efficiency in the step (A2), a sufficiently reduced content of insulin, IGF and the like in a culture medium is known to be important. For this reason, a serum free medium or a low serum culture medium is preferably used in the step (A2). The concentration of serum is preferably 0 to 3% (v/v), more preferably 0 to 2% (v/v).

In a preferred embodiment, a low serum RPMI1640 culture medium is used which is supplemented with activin A, CHIR99021.

The culturing period in the step (A2) is, for example, 1 to 3 days, preferably 1 to 2 days.

### (Step (B2))

In the step (B2), the cell obtained in the step (A2) is cultured in the presence of a growth factor belonging to the TGF-β superfamily.

Growth factors belonging to the TGF-β superfamily include activin, nodal, BMP and the like, and among these, activin is preferred. Examples of activin include activin A, activin B, activin AB and the like, and among these, activin A is preferred.

The concentration of a growth factor belonging to the TGF-β superfamily is preferably 5 to 250 ng/mL, more preferably 10 to 200 ng/mL and even more preferably 50 to 150 ng/mL.

A container and a culture medium used for culture may be the same as those used in the step (A2). That is, in a preferred embodiment, a low serum RPMI1640 culture medium is used which is supplemented with activin A.

The culturing period in the step (B2) is, for example, 1 to 4 days, preferably 1 to 3 days.

### (Step (C2))

In the step (C2), the cell obtained in the step (B2) is cultured in the presence of retinoid.

Examples of retinoid include retinol, retinal, retinoic acid and the like, and among these, retinoic acid is preferred.

The concentration of retinoid is preferably 0.2 to 10 µM, more preferably 0.4 to 8 µM and even more preferably 1 to 4 µM.

Further, in the step (C2), a BMP receptor inhibitor is preferably added to a culture medium.

BMP acceptor inhibitors include dorsomorphin (6-[4-[2-(1-piperidinyl)ethoxy]phenyl]-3-(4-pyridyl)pyrazolo[1,5-a]pyrimidine), LDN-193189 (4-(6-(4-(piperazine-1-yl)phenyl)pyrazolo[1,5-a]pyrimidine-3-yl)quinoline) and the like, and among these, dorsomorphin is preferred.

The concentration of a BMP acceptor inhibitor is preferably 0.2 to 5 µM, more preferably 0.3 to 3 µM and even more preferably 0.5 to 2 µM.

Further, in the step (C2), a TGF-β1 type receptor inhibitor is preferably added to a culture medium.

TGF-β1 type acceptor inhibitors include SB431542 (4-[4-(1,3-benzodioxol-5-yl)-5-(2-pyridinyl)-1H-imidazole-2-yl]benzamide), SB525334 (6-[2-(1,1-dimethylethyl)-5-(6-methyl-1,2-pyridinyl)-1H-imidazole-4-yl]quinoxaline), LY364947 (4-[3-(2-pyridinyl)-1H-pyrazole-4-yl]quinoline) and the like, and among these, SB431542 is preferred. Further, as a TGF-β 1 type acceptor inhibitor, Alk5 inhibitor II from Calbiochem may also be used.

The concentration of a TGF-β 1 type acceptor inhibitor is preferably 1 to 50 µM, more preferably 2 to 30 µM and even more preferably 5 to 20 µM.

A container used for culture may be the same as that used in the step (B2). A culture medium may be basically the same as that used in the step (B2) except for each of the aforementioned factors. However, instead of serum, a serum substitute is preferably added to the culture medium. As commercial products of a serum substitute, a B-27™ supplement, a N-2 supplement, a Knockout™ serum substitute from Invitrogen and the like are available, and among these, the B-27™ supplement is preferred.

The concentration of a B-27™ supplement is preferably 0.1 to 10% (v/v), more preferably 0.2 to 5% (v/v) and even more preferably 0.4 to 2.5% (v/v). Note that the B-27™ supplement, which is commercially available as a 50x stock solution, may be added to the culture medium such that it is diluted by 5 to 500 times in order to give a concentration of the B-27™ supplement of 0.1 to 10% (v/v).

In a preferred embodiment, a serum free IMEM Zinc Option culture medium is used which is supplemented with retinoic acid, dorsomorphin, SB431542, a B-27™ supplement.

The culturing period in the step (C2) is, for example, 5 to 9 days, preferably 6 to 8 days.

### (Step (D2))

In the step (D2), the cell obtained in the step (C2) is cultured in the presence of at least one factor selected from the group consisting of a cAMP increasing agent, dexamethasone, a TGF-β1 type acceptor inhibitor and nicotinamide.

With regards to a cAMP increasing agent, dexamethasone, a TGF-β 1 type acceptor inhibitor and nicotinamide, 2 or more of them are preferably added, and more preferably 3 or more are added.

cAMP increasing agents include an adenylate cyclase activator such as forskolin; a phosphodiesterase inhibitor such as 3-isobutyl-1-methylxanthin; a cAMP analog such as dibutyryl cAMP; and the like, and among these, forskolin is preferred.

The concentration of a cAMP increasing agent is preferably 1 to 50 µM, more preferably 2 to 30 µM and even more preferably 5 to 20 µM.

The concentration of dexamethasone is preferably 1 to 50 µM, more preferably 2 to 30 µM and even more preferably 5 to 20 µM.

TGF-β 1 type acceptor inhibitors include SB431542, SB525334, LY364947 and the like, and among these, SB431542 is preferred. Further, as a TGF-β 1 type acceptor inhibitor, Alk5 inhibitor II from Calbiochem may also be used.

The concentration of a TGF-β 1 type acceptor inhibitor is preferably 1 to 50 µM, more preferably 2 to 30 µM and even more preferably 5 to 20 µM.

The concentration of nicotinamide is preferably 1 to 30 mM, more preferably 3 to 20 mM and even more preferably 5 to 15 mM.

A container and culture medium used for culture may be the same those used in the step (C2). That is, a serum substitute is preferably added to a culture medium.

In a preferred embodiment, a serum free IMEM Zinc Option culture medium is used which is supplemented with forskolin, dexamethasone, Alk5 inhibitor II, nicotinamide, a B-27™ supplement.

The culturing period in the step (D2) is, for example, 9 to 13 days, more preferably 10 to 12 days.

In this step (D2), a pancreatic hormone-producing cell can be obtained.

Progress of differentiation-inducing into a pancreatic hormone-producing cell can be evaluated by observing production of pancreatic hormones such as insulin, glucagon, somatostatin, or alternatively evaluated by observing gene expression using RT-PCR. As differentiation of a pluripotent stem cell into a pancreatic hormone-producing cell progresses, expression of at least one of marker genes for a pancreatic hormone-producing cell INS, GCG, GHRL, SST, PPY and the like is promoted.

### Method for inducing differentiation of a pancreas tissue stem/precursor cell into a pancreatic hormone-producing cell (method of producing pancreatic hormone-producing cell)

In the method of producing a pancreatic hormone-producing cell described herein, the aforementioned differentiation-inducing promoter is added to a culture medium in a differentiation-inducing process from a pancreas tissue stem/precursor cell into the pancreatic hormone-producing cell. There is no particular limitation for the method for inducing differentiation of a pancreas tissue stem/precursor cell into a pancreatic hormone-producing cell, and any publicly known methods can be used. When adding a polypeptide or a modified polypeptide as a differentiation-inducing promoter, the concentration thereof is preferably 10 to 200 ng/mL, more preferably 50 to 150 ng/mL and even more preferably 60 to 120 ng/mL. Further, when adding a culture supernatant as a differentiation-inducing promoter, the concentration thereof is preferably 0.5 to 20% (v/v), more preferably 1 to 10% (v/v) and even more preferably 1.5 to 5% (v/v).

Below, an example of methods for inducing differentiation of a pancreas tissue stem/precursor cell into a pancreatic hormone-producing cell (a method of producing a pancreatic hormone-producing cell) will be described. However, the method of producing a pancreatic hormone-producing cell disclosed herein is not limited to this example.

A differentiation-inducing method described below is performed in accordance with the method described in Nonpatent Literature 2.

This differentiation-inducing method comprises the following steps (A3) to (E3). In at least one of these steps, the aforementioned differentiation-inducing promoter is added to a culture medium. The step of adding a differentiation-inducing promoter is preferably performed in at least one of the steps (D3) to (E3), and in particular preferably in the step (E3). Note that when adding a differentiation-inducing promoter in a certain step, it may be added at the beginning of the step or may be added in the middle of the step.
(A3) the step of culturing a pancreas tissue stem/precursor cell in the absence of a growth factor belonging to the TGF-β superfamily, retinoid, FGF and nicotinamide.
(B3) the step of culturing the cell obtained in the step (A3) in the presence of a growth factor belonging to the TGF-β super family.
(C3) the step of culturing the cell obtained in the step (B3) in the presence of retinoid.
(D3) the step of culturing the cell obtained in the step (C3) in the presence of FGF.
(E3) the step of culturing the cell obtained in the step (D3) in the presence of nicotinamide.

### (Step (A3))

In the step (A3), a pancreas tissue stem/precursor cell is cultured in the absence of a growth factor belonging to the TGF-β superfamily, retinoid, FGF, nicotinamide.

For a container used for culture, a culture plate coated with a scaffold of a biocompatible material is preferred in view of differentiation-inducing ability, functional expression ability, viability and the like. Scaffolds include laminin, fibronectin, collagen, heparan sulphate proteoglycan, gelatin, entactin, polyornithine and the like. As commercially available products, MATRIGEL™, growth factor reduced MATRIGEL™ from Becton Dickinson and the like are available. In particular, a culture plate coated with MATRIGEL™ is preferably used.

A culture medium used for culture is prepared by adding various nutrient sources and other components required for maintaining cell growth to a basal medium which can be used for culturing an animal cell.

Basal media include a RPMI1640 culture medium, a DMEM culture medium, a CMRL1066 culture medium, Ham's F12 culture medium, an Eagle MEM culture medium, a Glasgow MEM culture medium, an IMEM Zinc Option culture medium, an IMDM culture medium, a William E culture medium, a Fischer culture medium, a McCoy culture medium, a BME culture medium, an αMEM culture medium, a BGJb culture medium, a Medium 199 culture media, or mixed media thereof and the like.

Nutrient sources include carbon sources such as glycerol, glucose, fructose, sucrose, lactose, starch, dextrin; hydrocarbons such as fatty acid, fat and oil, lecithin, alcohol; nitrogen sources such as ammonium sulfate, ammonium nitrate, ammonium chloride, urea, sodium nitrate: inorganic salts such as sodium salts, potassium salts, magnesium salts, calcium salts, phosphates; various vitamins; various amino acids; and the like.

Other components include antibiotics such as penicillin, streptomycin; cholera toxin; insulin; transferrin; selenious acid; 2-mercaptoethanol; albumin; serum or serum substitutes; and the like. With regards to insulin, transferrin and selenious acid, ITS-X, ITS-A, ITS-G from Invitrogen and the like are available as commercial products. Further, as serum substitutes, a B-27™ supplement, a N-2 supplement, a Knockout™ serum substitute from Invitrogen and the like are available as commercial products.

In a preferred embodiment, a serum free DMEM/Ham's F12 is used which is supplemented with antibiotics such as penicillin, streptomycin; insulin; transferrin; selenious acid; 2-mercaptoethanol; albumin.

The concentration of insulin is preferably 2 to 30 µg/mL, more preferably 5 to 20 µg/mL. The concentration of transferrin is preferably 1 to 20 µg/mL, more preferably 3 to 10 µg/mL. The concentration of selenious acid is preferably 1 to 20 ng/mL, more preferably 5 to 20 ng/mL. The concentration of 2-mercaptoethanol is preferably 50 to 200 µM, more preferably 50 to 100 µM. The concentration of albumin is preferably 1 to 10 ng/mL, more preferably 2 to 5 ng/mL.

The culturing period in the step (A3) is 1 to 3 days, preferably 1 to 2 days.

### (Step (B3))

In the step (B3), the cell obtained in the step (A3) is cultured in the presence of a growth factor belonging to the TGF-β superfamily.

Growth factors belonging to the TGF-β superfamily include activin, nodal, BMP and the like, and among these, activin is preferred. Examples of activin include activin A, activin B, activin AB and the like, and among these, activin A is preferred.

The concentration of a growth factor belonging to the TGF-β superfamily is preferably 5 to 250 ng/mL, more preferably 10 to 200 ng/mL and even more preferably 50 to 150 ng/mL.

A container used for culture may be the same as that used in the step (A3). A culture medium may be the same as that used in the step (A3) except that a growth factor belonging to the TGF-β superfamily is added. That is, in a preferred embodiment, a serum free DMEM/Ham's F12 is used which is supplemented with antibiotics such as penicillin, streptomycin; insulin, transferrin, selenious acid, 2-mercaptoethanol, albumin.

The culturing period in the step (B3) is 2 to 6 days, preferably 3 to 5 days.

### (Step (C3))

In the step (C3), the cell obtained in the step (B3) is cultured in the presence of retinoid.

Examples of retinoid include retinol, retinal, retinoic acid and the like, and among these, all-trans-retinoic acid is preferred.

The concentration of retinoid is preferably 0.2 to 10 µM, more preferably 0.4 to 8 µM and even more preferably 1 to 4 µM.

A container used for culture may be the same as that used in the step (A3). A culture medium may be the same as that used in the step (A3) except that a growth factor belonging to the TGF-β superfamily is added.

In a preferred embodiment, a serum free DMEM/Ham's F12 is used which is supplemented with all-trans-retinoic acid, antibiotics such as penicillin, streptomycin, insulin, transferrin, selenious acid, 2-mercaptoethanol, albumin.

The culturing period in the step (C3) is, for example, 2 to 6 days, preferably 3 to 5 days.

### (Step (D3))

In the step (D3), the cell obtained in the step (C3) is cultured in the presence of FGF.

Examples of FGF include FGF-1, FGF-2 (bFGF), FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, FGF-23 and the like, and FGF-2 (bFGF), FGF-5, FGF-7, FGF-10 are preferred.

The concentration of FGF is preferably 1 to 30 ng/mL, more preferably 2 to 20 ng/mL and even more preferably 5 to 15 ng/mL.

A container used for culture may be the same as that used in the step (A3). A culture medium may be basically the same as that used in the step (C3) except that FGF is added.

In a preferred embodiment, a serum free DMEM/Ham's F12 is used which is supplemented with FGF-2 (bFGF), antibiotics such as penicillin, streptomycin, insulin, transferrin, selenious acid, albumin.

The culturing period in the step (D3) is, for example, 1 to 5 days, preferably 2 to 4 days.

### (Step (E3))

In the step (E3), the cell obtained in the step (D3) is cultured in the presence of nicotinamide.

The concentration of nicotinamide is preferably 1 to 30 mM, more preferably 3 to 20 mM and even more preferably 5 to 15 mM.

Further, in the step (E3), FGF is preferably added to a culture medium.

Examples of FGF include FGF-1, FGF-2 (bFGF), FGF-3, FGF-4, FGF-5, FGF-6, FGF-7, FGF-8, FGF-9, FGF-10, FGF-11, FGF-12, FGF-13, FGF-14, FGF-15, FGF-16, FGF-17, FGF-18, FGF-19, FGF-20, FGF-21, FGF-22, FGF-23 and the like, and FGF-2 (bFGF), FGF-5, FGF-7, FGF-10 are preferred.

The concentration of FGF is preferably 1 to 30 ng/mL, more preferably 2 to 20 ng/mL and even more preferably 5 to 15 ng/mL.

A container used for culture may be the same as that used in the step (A3). A culture medium may be basically the same as that used in the step (D3) except that nicotinamide is added.

In a preferred embodiment, a serum free DMEM/Ham's F12 is used which is supplemented with nicotinamide, FGF-2 (bFGF), antibiotics such as penicillin, streptomycin, insulin, transferrin, selenious acid, albumin.

The culturing period in the step (E3) is, for example, 3 to 20 days, preferably 3 to 10 days.

In this step (E3), a pancreatic hormone-producing cell can be obtained.

Progress of differentiation-inducing into a pancreatic hormone-producing cell can be evaluated by observing production of pancreatic hormones such as insulin, glucagon, somatostatin, or alternatively evaluated by observing gene expression using RT-PCR. As differentiation of a pancreas tissue stem/precursor cell into a pancreatic hormone-producing cell progresses, expression of at least one of marker genes for a pancreatic hormone-producing cell INS, GCG, GHRL, SST, PPY and the like is promoted.

### Example of application of pancreatic hormone-producing cell

A pancreatic hormone-producing cell obtained as described above can be used as a therapeutic agent for diabetes and the like. For example, in a case where pancreatic hormone-producing cells produce/secrete insulin, the insulin-producing cells can be used as a therapeutic agent for diabetes without further processing or in a form of cell clumps such as pellets prepared by concentrating through a filter. This therapeutic agent for diabetes can also be cryopreserved after adding a protective agent such as DMSO. Note that for safer use, the agent is preferably treated under conditions such that pathogenic proteins are denatured while functions as a therapeutic agent for diabetes is preserved, the treatment including heat treatment, radiation treatment, mitomycin C treatment.

Modes of administration of insulin-producing cells as a therapeutic agent for diabetes (methods of implantation) to human include: for example, a method comprising providing a small incision in the lower right abdomen of a patient, exposing a thin blood vessel in the mesentery, and inserting a catheter to implant the cells under direct vision; a method comprising identifying the portal vein in liver by echo, performing paracentesis with a catheter to implant the cells; or a method comprising directly making a puncture in the spleen under the guide of abdomen echo for implantation in the spleen. The dosage (the amount of transplantation) is preferably 1x10⁸ to 1x10¹⁰ cells/individual, more preferably 5x10⁸ to 1x10¹⁰ cells/individual. Note that the dosage (the amount of transplantation) may be appropriately altered depending on age, weight, conditions and the like of a patient to be administered.

Further, the pancreatic hormone-producing cell obtained as described above can be used as a research reagent. For example, screening for a new drug can be performed by adding the new drug to a culture container in which a pancreatic hormone-producing cell is cultured, or to a bioreactor in which a pancreatic hormone-producing cell is confined.

Further, it is also possible to manufacture a bioartificial pancreas using the pancreatic hormone-producing cells obtained as described above. Examples of a bioartificial pancreas include a hybrid artificial pancreas in which a hollow fiber bioreactor (device) is combined with pancreatic hormone-producing cells. Examples of a bioartificial pancreas include those attached outside the body and connected to a blood vessel, those placed inside the body and connected to a blood vessel, those placed intraperitoneally but not connected to a blood vessel, those placed subcutaneously but not connected to a blood vessel. They are applicable to any types of a bioartificial pancreas.

### EXAMPLES

Below, the present invention will be described in detail with reference to Examples, but the present invention will not be in any way construed as limited to the following description.

### Example 1

### (1) Preparation of differentiation-inducing promoter

A differentiation-inducing promoter was prepared as follows. Onto a 10-cm dish, plated were 5x10⁵ CHO-K1 cells subcultured in Ham's F12 culture medium (Sigma, N6658) supplemented with 10% FBS (fetal bovine serum) (NICHIREI CORPORATION, 171012), 1% penicillin/streptomycin (Life Technologies Japan, 15140-122). On the following day, the CHO-K1 cells were transfected with an expression vector (pCAGGS-IBCAP) for a polypeptide encoded by DNA consisting of the base sequence set forth in SEQ ID NO.1 (hereinafter referred to as "IBCAP") using FuGENE6 (Roche). After 48 hours, the cells were diluted to a concentration of 1/20, and again plated onto a 10-cm dish. On the following day, G418 (NACALAI TESQUE, INC., 09380-44) was added at a final concentration of 400 µg/mL, and colonies were allowed to form with a culture medium replaced every 3 to 5 days. A colony cloned by limiting dilution was isolated and proliferated, and then gene expression was determined by Southern blotting and Northern blotting to prepare a CHO-K1 cell strain stably expressing IBCAP (hereinafter referred to as "IBCAP expressing stable CHO cells") .

Then the IBCAP expressing stable CHO cells were conditioned in CD OptiCHO (Life Technologies Japan, 12681-011) supplemented with 1 % GLUTAMAX I (Life Technologies Japan, 35050-061) and 1% penicillin/streptomycin (Life Technologies Japan, 15140-122). Further, the conditioned IBCAP expressing stable CHO cells were cultured with shaking in a forced aeration CO₂ incubator (TAITEC CORPORATION, CO₂-BR-43FL, temperature: 37°C, shaking speed: 120 rpm, gas conditions: 5% CO₂, 20 mL/min/flask). Then, a culture supernatant was prepared using these cells afterwards.

The culture supernatant, which was determined to show a survival rate of 90% or more, was subcultured to give a cell count of 5x10⁵ cells/mL (the amount of culture medium: 150 mL culture medium/500 mL flask), and then collected after 5 days (the cell count was about 4 to 5x10⁵ cells/mL). The culture supernatant collected was then concentrated about 10 times using a Centriprep (Millipore, 4302, YM-3) (300 mL was concentrated to about 30 mL), and further dialyzed 3 times against 2 L of 30 mM HEPES (pH 7.6). Then the culture supernatant after dialysis (hereinafter, referred to as a "IBCAP culture supernatant") was prepared as a differentiation-inducing promoter.

Furthermore, as a mock control, CHO-K1 cells were transfected with an empty vector (pCAGGS), and a culture supernatant after dialysis (hereinafter, referred to as a "mock culture supernatant") was prepared as described above.

### (2) Inducing differentiation of human iPS cell into pancreatic hormone-producing cell

With regards to human iPS cells, the TIG3/KOSM cells provided by Dr. Mitani at Saitama Medical School were used. The cells have been established at the National Institute of Advanced Industrial Science and Technology by introducing four factors (the OCT gene, the KLF gene, the SOX gene, the MYC gene) into TIG-3 cells using Sendai Virus (Nishimura, K. et al., J. Biol. Chem., 286, pp. 4760-4771 (2011)). The TIG3/KOSM cells were maintained in a DMEM/Ham's F12 culture medium supplemented with 1% (v/v) penicillin/streptomycin (Gibco), 20% (v/v) Knockout™ serum substitute (Gibco), 1% (v/v) non-essential amino acids (Gibco), 2.5 mM L-glutamine, 0.1 mM 2-mercaptoethanol (Gibco), 5 ng/mL FGF-2 (R&D Systems), 5 mM sodium chloride.

One day before the start of differentiation inducing, the TIG3/KOSM cells grown on a 10-cm dish were plated into 6-well plate coated with MATRIGEL™ (Becton Dickinson) at a cell density of 1x10⁹ cells/well, and cultured overnight in a culture medium conditioned with STO feeder cells. Then, the culture medium was removed, and 1 mL CTK (0.25% trypsin, 1 mg/mL Collagenase IV, 20% KSR, 1 mM CaCl₂ in PBS) was added and treated at 37°C for 5 minutes. After STO feeder cells were removed, TIG3/KOSM cells were detached by suspension with pipetting. Then, the detached TIG3/KOSM cells were transferred into a 15 mL tube, and centrifuged at 1000 rpm (150xg) for 5 minutes, and then a supernatant was removed. Subsequently, the cells were plated into a 6-well plate coated with Matrigel™ at a cell density of 1x10⁹ cells/well.

Note that the conditioned medium described above was prepared as follows. That is, 7.5x10⁶ cells of mitomycin treated STO cells were plated onto a 15-cm dish. On the following day, the culture medium was replaced with a human iPS culture medium (without FGF-2), and treated for 1 to 3 hours, and then the culture medium was again replaced with a human iPS culture medium, and cultured for 24 hours. On the following day, the supernatant was collected, and cells were removed by centrifugation at 1500 rpm (330xg) for 10 minutes, and stored at -20 to -30°C.

On the first day of starting induction of differentiation, the culture medium was replaced with an Advanced RPMI1640 culture medium (Gibco) supplemented with 1% (v/v) penicillin/streptomycin (Gibco), 2 mM L-glutamine (Gibco), 100 ng/mL activin A (Humanzyme), 25 ng/mL Wnt3a (NACALAI TESQUE, INC.), and cultured for 1 day (Step (A1-1)). Next, the culture medium was replaced with an Advanced RPMI1640 culture medium (Gibco) supplemented with 1% (v/v) penicillin/streptomycin (Gibco), 0.2% (v/v) FBS (fetal bovine serum), 2 mM L-glutamine (Gibco), 100 ng/mL activin A (Gibco), and cultured for 2 days (Step (A1-2)).

Next, the culture medium was replaced with an Advanced RPMI1640 culture medium (Gibco) supplemented with 1% (v/v) penicillin/streptomycin (Gibco), 2% (v/v) FBS, 2 mM L-glutamine (Gibco), 50 ng/mL FGF-10 (R&D Systems), 0.25 µM KAAD-cyclopamine (NACALAI TESQUE, INC.), and cultured for 2 days (Step (B1)).

Next, the culture medium was replaced with a DMEM/Ham's F12 culture medium (Gibco) supplemented with 1% (v/v) penicillin/streptomycin (Gibco), 2% (v/v) B-27™ supplement (Gibco), 2 µM retinoic acid (Sigma), 0.25 µM KAAD-cyclopamine (NACALAI TESQUE, INC.), 50 ng/mL FGF-10 (R&D Systems), and cultured for 4 days (Step (C1)).

Next, the culture medium was replaced with a DMEM/Ham's F12 culture medium (Gibco) supplemented with 1% (v/v) penicillin/streptomycin (Gibco), 2% (v/v) B-27™ supplement (Gibco), 10 µM DAPT (Sigma), 55 nM exendin-4 (Phoenix Pharmaceuticals), and further supplemented with 2% (v/v) IBCAP culture supernatant or mock culture supernatant, and cultured for 3 days (Step (D1)).

Finally, the culture medium was replaced with a CMRL1066 culture medium (Gibco) supplemented with 1% (v/v) penicillin/streptomycin (Gibco), 2% (v/v) B-27™ supplement (Gibco), 55 nM exendin-4 (Phoenix Pharmaceuticals), 50 ng/mL HGF (Humanzyme), 50 ng/mL IGF-1 (Humanzyme), and further supplemented with 2% IBCAP culture supernatant or mock culture supernatant, and cultured for 6 days (Step (E1)).

### (3) Quantitative RT-PCR analysis

Gene expressions of glucagon (GCG) and somatostatin (SST) were determined by quantitative RT-PCR for the TIG3/KOSM cells before inducing differentiation and the cells obtained via the step (E1). Specifically, first, RNA was extracted from cells using NucleoSpin™ RNA II (TAKARA BIO INC.), and quantitative RT-PCR analysis was performed using Fast SYBR™ Green PCR MasterMix (Applied Biosystems). Primer sequences are shown below. HsGCG_264F: GCATTTACTTTGTGGCTGGA (SEQ ID NO. 4)
HsGCG_368R: CCTGGGAAGCTGAGAATGAT (SEQ ID NO. 5)
HsSST_206F: CCCCAGACTCCGTCAGTTTC (SEQ ID NO. 6)
HsSST_313R: TCCGTCTGGTTGGGTTCAG (SEQ ID NO. 7)

PCR products were separated by 3% agarose gel electrophoresis, and visualized with ethidium bromide, BioDoc-It Imaging System (BMbio).

The expression levels of glucagon (GCG) and somatostatin (SST) in the cells obtained via the step (E1) are shown in Fig. 1 (a) and (b), respectively. Fig. 1 (a) and (b) show relative values (fold induction) when the expression levels of glucagon (GCG) and somatostatin (SST) in the TIG3/KOSM cells before inducing differentiation were taken as 1.

As shown in Fig. 1 (a), the fold induction of glucagon (GCG) was about 22.6 fold when nothing was added whereas it was about 12.8 fold when a mock culture supernatant was added in the steps (D1), (E1), and about 36.8 fold when an IBCAP culture supernatant was added.

Moreover, as shown in Fig. 1 (b), the fold induction of somatostatin (SST) was almost 0 fold when nothing was added whereas it was about 0.4 fold when a mock culture supernatant was added in the steps (D1), (E1), and about 9.5 fold when an IBCAP culture supernatant was added.

These results reveal that differentiation inducing efficiency of a human iPS cell into a pancreatic hormone-producing cell can be improved by adding an IBCAP culture supernatant in the steps (D1), (E1).

### Example 2

TIG3/KOSM cells were cultured as in Example 1 except that an IBCAP culture supernatant or a mock culture supernatant was added in the steps (A1-1), (A1-1) or the steps (B1), (C1), and the culturing period in the step (E1) was 3 days. Gene expressions of glucagon (GCG) and somatostatin (SST) were then determined by quantitative RT-PCR.

The expression levels of glucagon (GCG) and somatostatin (SST) in the cells obtained via the step (E1) are shown in Fig. 2 (a) and (b), respectively. Fig. 2 (a), (b) show relative values (fold induction) when the expression levels of glucagon (GCG) and somatostatin (SST) in the TIG3/KOSM cells before inducing differentiation were taken as 1.

As shown in Fig. 2 (a), the fold induction of glucagon (GCG) was about 120.1 fold when nothing was added whereas it was about 100.7 fold when a mock culture supernatant was added in the steps (A1-1), (A1-2), and about 193.8 fold when an IBCAP culture supernatant was added. Further, it was about 31.2 fold when a mock culture supernatant was added in the steps (B1), (C1), and about 218.5 fold when an IBCAP culture supernatant was added.

Moreover, as shown in Fig. 2 (b), the fold induction of somatostatin (SST) was about 117.6 fold when nothing was added whereas it was about 16.6 fold when a mock culture supernatant was added in the steps (A1-1), (A1-2), and about 65.2 fold when an IBCAP culture supernatant was added in the steps (A1-1), (A1-2). Moreover, it was about 8.8 fold when a mock culture supernatant was added in the steps (B1), (C1), and about 164.1 fold when an IBCAP culture supernatant was added.

These results reveal that differentiation-inducing efficiency of a human iPS cell into a pancreatic hormone-producing cell can be improved either by adding an IBCAP culture supernatant in the steps (A1-1), (A1-2) or by adding an IBCAP culture supernatant in the steps (B1), (C1).

### Example 3

### (1) Inducing differentiation of mouse pancreas tissue stem/precursor cell into pancreatic hormone-producing cell

With regards to mouse pancreas tissue stem/precursor cells, Tec3DR cells provided by Dr. Matsumoto at Saitama Medical School were used. The cells have been established by cloning a tissue stem/precursor cell separated from mouse pancreas in the fetal period. Tec3DR cells were maintained in a DMEM culture medium supplemented with 1% (v/v) penicillin/streptomycin (Gibco), 15% (v/v) FBS, 50 µM 2-mercaptoethanol (Gibco).

On the first day of starting induction of differentiation, Tec3DR cells were plated onto 24-well plate at a cell density of 1x10⁵ cells/well, and cultured for 2 days in a DMEM/Ham's F12 culture medium (Gibco) supplemented with 1% (v/v) penicillin/streptomycin (Gibco), 0.1% (v/v) BSA (bovine serum albumin) (Sigma), 1% (v/v) ITS-X (Gibco), 55 µM 2-mercaptoethanol (Gibco) (Step (A3)).

Next, the culture medium was replaced with a DMEM/Ham's F12 culture medium (Gibco) supplemented with 1% (v/v) penicillin/streptomycin (Gibco), 0.1% (v/v) BSA (Sigma), 1% (v/v) ITS-X (Gibco), 55 µM 2-mercaptoethanol (Gibco), 50 ng/mL activin A (Humanzyme), and cultured for 4 days (Step (B3)).

Next, the culture medium was replaced with a DMEM/Ham's F12 culture medium (Gibco) supplemented with 1% (v/v) penicillin/streptomycin (Gibco), 0.1% (v/v) BSA (Sigma), 1% (v/v) ITS-X (Gibco), 55 µM 2-mercaptoethanol (Gibco), 1 µM retinoic acid (Sigma), and cultured for 4 days (Step (C3)).

Next, the cells in culture were collected by trypsin-EDTA treatment, and plated into 24-well plate at a cell density of 1x10⁵ cells/well, and cultured for 3 days in a DMEM/Ham's F12 culture medium (Gibco) supplemented with 1% (v/v) penicillin/streptomycin (Gibco), 2 mg/mL BSA (Sigma), 1% (v/v) ITS-X (Gibco), 10 ng/mL FGF-2 (NACALAI TESQUE, INC.) (Step (D3)) .

Finally, the culture medium was replaced with a DMEM/Ham's F12 culture medium (Gibco) supplemented with 1% (v/v) penicillin/streptomycin (Gibco), 2 mg/mL BSA (Sigma), 1% (v/v) ITS-X (Gibco), 10 ng/mL FGF-2 (NACALAI TESQUE, INC.), 10 mM nicotinamide (Sigma), and cultured for 5 days. During this, the culture medium was replaced with a fresh culture medium after 2 to 3 days. Then, 2% (v/v) IBCAP culture supernatant or mock culture supernatant was added to the culture medium, and further cultured for 6 days (Step (E3)).

### (2) Quantitative RT-PCR analysis

Gene expression of mouse insulin 1 (Ins1) was determined by quantitative RT-PCR for the Tec3DR cells before inducing differentiation and the cells obtained via the step (E3). Specifically, first, RNA was extracted from cells using NucleoSpin™ RNA II (TAKARA BIO INC.), and quantitative RT-PCR analysis was performed using Power SYBR™ Green PCR Master Mix (Applied Biosystems). Primer sequences are shown below.
MnIns1_qPCR_Fw: CACTTCCTACCCCTGCTGG (SEQ ID NO. 8)
MnIns1_qPCR_Rv: ACGCCAAGGTCTGAAGGTC (SEQ ID NO. 9)

PCR products were separated by 3% agarose gel electrophoresis, stained with ethidium bromide, and visualized with BioDoc-It Imaging System (BMbio).

Fig. 3 shows the expression level of insulin-1 (Ins1) in the cells obtained via the step (E3). Fig. 3 shows relative values when the expression level of insulin-1 (Ins1) in the Tec3DR cells before inducing differentiation is taken as 1.

As shown in Fig. 3, it was about 0.3 fold when a mock culture supernatant was added in the step (E3), and it was about 17.0 fold when an IBCAP culture supernatant was added.

These results reveal that differentiation-inducing efficiency of a mouse pancreas tissue stem/precursor cell into a pancreatic hormone-producing cell can be improved by adding an IBCAP culture supernatant in the step (E3).

## Claims

1. A method of producing a pancreatic hormone-producing cell from a pluripotent stem cell or a pancreas tissue stem/precursor cell, the method comprising the steps of:
(A1) culturing the pluripotent stem cell in the presence of a growth factor belonging to the TGF-β (transforming growth factor β) superfamily,
(B1) culturing the cell obtained in the step (A1) in the presence of FGF (fibroblast growth factor),
(C1) culturing the cell obtained in the step (B1) in the presence of retinoid,
(D1) culturing the cell obtained in the step (C1) in the presence of a γ-secretase inhibitor and
(E1) culturing the cell obtained in the step (D1) in the presence of at least one factor selected from the group consisting of exendin-4, HGF (hepatocyte growth factor), IGF-1 (insulin like growth factor-1) and nicotinamide,
wherein at least one of the steps (A1) to (E1) is a differentiation-inducing process from the pluripotent stem cell or the pancreas tissue stem/precursor cell to the pancreatic hormone-producing cell, and
at least one differentiation-inducing promoter selected from (1) to (3) is added to a culture medium in at least one of the steps (A1) to (E1):
(1) a polypeptide consisting of an amino acid sequence encoded by DNA consisting of the base sequence set forth in SEQ ID NO. 1,
(2) a polypeptide consisting of an amino acid sequence having one or several amino acid substitutions, deletions, and/or additions in the amino acid sequence encoded by DNA consisting of the base sequence set forth in SEQ ID NO. 1, and having a differentiation-inducing promotion effect into a pancreatic hormone-producing cell,
(3) a culture supernatant of cells into which DNA consisting of the base sequence set forth in SEQ ID NO.1 or DNA hybridizing with DNA consisting of a base sequence complementary to the DNA consisting of the base sequence set forth in SEQ ID NO: 1 under stringent conditions is incorporated as a foreign gene, wherein the DNA hybridizing with DNA consisting of the base sequence complementary to the DNA consisting of the base sequence set forth in SEQ ID NO: 1 encodes a polypeptide having a differentiation-inducing promotion effect into a pancreatic hormone-producing cell.

2. A method of producing a pancreatic hormone-producing cell from a pluripotent stem cell or a pancreas tissue stem/precursor cell, the method comprising the steps of:
(A2) culturing the pluripotent stem cell in the presence of at least one factor selected from the group consisting of a growth factor belonging to the TGF-β (transforming growth factor β) superfamily and a growth factor belonging to the Wnt (wingless type MMTV integration site) family and a GSK-3 (glycogen synthase kinase-3) inhibitor,
(B2) culturing the cell obtained in the step (A2) in the presence of a growth factor belonging to the TGF-β super family,
(C2) culturing the cell obtained in the step (B2) in the presence of retinoid,
(D2) culturing the cell obtained in the step (C2) in the presence of at least one factor selected from the group consisting of a cAMP (cyclic adenosine monophosphate) increasing agent, dexamethasone, a TGF-β1 type acceptor inhibitor and nicotinamide,
wherein at least one of the steps (A2) to (D2) is a differentiation-inducing process from the pluripotent stem cell or the pancreas tissue stem/precursor cell to the pancreatic hormone-producing cell, and
at least one differentiation-inducing promoter selected from (1) to (3) is added to a culture medium in at least one of the steps (A2) to (D2):
(1) a polypeptide consisting of an amino acid sequence encoded by DNA consisting of the base sequence set forth in SEQ ID NO. 1,
(2) a polypeptide consisting of an amino acid sequence having one or several amino acid substitutions, deletions, and/or additions in the amino acid sequence encoded by DNA consisting of the base sequence set forth in SEQ ID NO. 1, and having a differentiation-inducing promotion effect into a pancreatic hormone-producing cell,
(3) a culture supernatant of cells into which DNA consisting of the base sequence set forth in SEQ ID NO.1 or DNA hybridizing with DNA consisting of a base sequence complementary to the DNA consisting of the base sequence set forth in SEQ ID NO: 1 under stringent conditions is incorporated as a foreign gene, wherein the DNA hybridizing with DNA consisting of the base sequence complementary to the DNA consisting of the base sequence set forth in SEQ ID NO: 1 encodes a polypeptide having a differentiation-inducing promotion effect into a pancreatic hormone-producing cell.

3. A method of producing a pancreatic hormone-producing cell from a pluripotent stem cell or a pancreas tissue stem/precursor cell, the method comprising the steps of:
(A3) culturing the pancreas tissue stem/precursor cell in the absence of a growth factor belonging to the TGF-β (transforming growth factor β) superfamily, retinoid, FGF (fibroblast growth factor) and nicotinamide,
(B3) culturing the cell obtained in the step (A3) in the presence of a growth factor belonging to the TGF-β super family,
(C3) culturing the cell obtained in the step (B3) in the presence of retinoid,
(D3) culturing the cell obtained in the step (C3) in the presence of FGF and
(E3) culturing the cell obtained in the step (D3) in the presence of nicotinamide,
wherein at least one of the steps (A3) to (E3) is a differentiation-inducing process from the pluripotent stem cell or the pancreas tissue stem/precursor cell to the pancreatic hormone-producing cell, and
at least one differentiation-inducing promoter selected from (1) to (3) is added to a culture medium in at least one of the steps (A3) to (E3):
(1) a polypeptide consisting of an amino acid sequence encoded by DNA consisting of the base sequence set forth in SEQ ID NO. 1,
(2) a polypeptide consisting of an amino acid sequence having one or several amino acid substitutions, deletions, and/or additions in the amino acid sequence encoded by DNA consisting of the base sequence set forth in SEQ ID NO. 1, and having a differentiation-inducing promotion effect into a pancreatic hormone-producing cell,
(3) a culture supernatant of cells into which DNA consisting of the base sequence set forth in SEQ ID NO.1 or DNA hybridizing with DNA consisting of a base sequence complementary to the DNA consisting of the base sequence set forth in SEQ ID NO: 1 under stringent conditions is incorporated as a foreign gene, wherein the DNA hybridizing with DNA consisting of the base sequence complementary to the DNA consisting of the base sequence set forth in SEQ ID NO: 1 encodes a polypeptide having a differentiation-inducing promotion effect into a pancreatic hormone-producing cell.

4. Use of a differentiation-inducing promoter for inducing differentiation of a pluripotent stem cell or a pancreas tissue stem/precursor cell into a pancreatic hormone-producing cell, comprising at least one of (1) to (3):
(1) a polypeptide consisting of an amino acid sequence encoded by DNA consisting of the base sequence set forth in SEQ ID NO. 1,
(2) a polypeptide consisting of an amino acid sequence having one or several amino acid substitutions, deletions, and/or additions in the amino acid sequence encoded by DNA consisting of the base sequence set forth in SEQ ID NO. 1, and having a differentiation-inducing promotion effect into a pancreatic hormone-producing cell,
(3) a culture supernatant of cells into which DNA consisting of the base sequence set forth in SEQ ID NO.1 or DNA hybridizing with DNA consisting of a base sequence complementary to the DNA consisting of the base sequence set forth in SEQ ID NO: 1 under stringent conditions is incorporated as a foreign gene, wherein the DNA hybridizing with DNA consisting of the base sequence complementary to the DNA consisting of the base sequence set forth in SEQ ID NO: 1 encodes a polypeptide having a differentiation-inducing promotion effect into a pancreatic hormone-producing cell.

## Patentansprüche

1. Verfahren zur Herstellung einer Pankreashormon-produzierenden Zelle aus einer pluripotenten Stammzelle oder einer Pankreas-Gewebestamm-/- Precursorzelle, wobei das Verfahren umfasst:
(A1) Kultivieren der pluripotenten Stammzelle in Gegenwart eines Wachstumsfaktors, der zu der TGF-β (Transformationswachstumsfaktor β) - Superfamilie gehört,
(B1) Kultivieren der in Schritt (A1) erhaltenen Zelle in Gegenwart von FGF (Fibroblastenwachstumsfaktor),
(C1) Kultivieren der in Schritt (B1) erhaltenen Zelle in Gegenwart von Retinoid,
(D1) Kultivieren der in Schritt (C1) erhaltenen Zelle in Gegenwart eines γ-Sekretase-Inhibitors und
(E1) Kultivieren der in Schritt (D1) erhaltenen Zelle in Gegenwart von mindestens einem Faktor, ausgewählt aus der Gruppe bestehend aus Exendin-4, HGF (Hepatozytenwachstumsfaktor), IGF-1 (insulinähnlicher Wachstumsfaktor-1) und Nicotinamid,
wobei mindestens einer der Schritte (A1) bis (E1) ein differenzierungsinduzierender Prozess von der pluripotenten Stammzelle oder der Pankreas-Gewebestamm-/-Precursorzelle zu der Pankreashormon-produzierenden Zelle ist, und
mindestens ein differenzierungsinduzierender Promotor, ausgewählt aus (1) bis (3) zu einem Kulturmedium in mindestens einem der Schritte (A1) bis (E1) gegeben wird:
(1) ein Polypeptid, bestehend aus einer Aminosäuresequenz, codiert von DNA, bestehend aus der Basensequenz, die in SEQ ID NO. 1 dargelegt ist,
(2) ein Polypeptid, bestehend aus einer Aminosäuresequenz, die eine oder mehrere Aminosäuresubstitutionen, -deletionen und / oder - additionen in der Aminosäuresequenz aufweist, die durch DNA codiert wird, bestehend aus der Basensequenz, die in SEQ ID NO. 1 dargelegt ist und eine differenzierungsinduzierende Promotionswirkung in einer Pankreashormon-produzierenden Zelle aufweist,
(3) einen Kulturüberstand von Zellen, in welche DNA, bestehend aus der Basensequenz, die in SEQ ID NO. 1 dargelegt ist oder DNA, die unter stringenten Bedingungen mit der DNA, bestehend aus der Basensequenz, die komplementär ist mit der Basensequenz, die in SEQ ID NO.1 dargelegt ist, hybridisiert, als ein fremdes Gen eingebaut wird, wobei die DNA, die mit der DNA, bestehend aus der Basensequenz, die komplementär ist mit der Basensequenz, die in SEQ ID NO.1 dargelegt ist, hybridisiert, für ein Polypeptid codiert, das eine differenzierungsinduzierende Promotionswirkung in einer Pankreashormon-produzierenden Zelle aufweist.

2. Verfahren zur Herstellung einer Pankreashormon-produzierenden Zelle aus einer pluripotenten Stammzelle oder einer Pankreas-Gewebestamm-/- Precursorzelle, wobei das Verfahren umfasst:
(A2) Kultivieren der pluripotenten Stammzelle in Gegenwart von mindestens einem Faktor, ausgewählt aus der Gruppe bestehend aus einem Wachstumsfaktor, der zu der TGF-β (Transformationswachstumsfaktor β) - Superfamilie gehört und einem Wachstumsfaktor, der zur Wnt-Familie (flankenlose [wingless] MMTV-Integrationsstelle) gehört, und einem GSK-3 (Glykogensynthase-Kinase-3) -Inhibitor,
(B2) Kultivieren der in Schritt (A2) erhaltenen Zelle in Gegenwart eines Wachstumsfaktors, der zu der TGF-β-Superfamilie gehört,
(C2) Kultivieren der in Schritt (B2) erhaltenen Zelle in Gegenwart von Retinoid,
(D2) Kultivieren der in Schritt (C2) erhaltenen Zelle in Gegenwart von mindestens einem Faktor, ausgewählt aus der Gruppe bestehend aus einem cAMP (zyklisches Adenosinmonophosphat)-erhöhenden Mittel, Dexamethason, einem Akzeptor-Inhibitor vom TGF-β1-Typ und Nicotinamid,
wobei mindestens einer der Schritte (A2) bis (D2) ein differenzierungsinduzierender Prozess von der pluripotenten Stammzelle oder der Pankreas-Gewebestamm-/-Precursorzelle zu der Pankreashormon-produzierenden Zelle ist, und
mindestens ein differenzierungsinduzierender Promotor, ausgewählt aus (1) bis (3) zu einem Kulturmedium in mindestens einem der Schritte (A2) bis (D2) gegeben wird:
(1) ein Polypeptid, bestehend aus einer Aminosäuresequenz, codiert von DNA, bestehend aus der Basensequenz, die in SEQ ID NO. 1 dargelegt ist,
(2) ein Polypeptid, bestehend aus einer Aminosäuresequenz, die eine oder mehrere Aminosäuresubstitutionen, -deletionen und / oder - additionen in der Aminosäuresequenz aufweist, die durch DNA codiert wird, bestehend aus der Basensequenz, die in SEQ ID NO. 1 dargelegt ist und eine differenzierungsinduzierende Promotionswirkung in einer Pankreashormon-produzierenden Zelle aufweist,
(3) einen Kulturüberstand von Zellen, in welche DNA, bestehend aus der Basensequenz, die in SEQ ID NO. 1 dargelegt ist oder DNA, die unter stringenten Bedingungen mit der DNA, bestehend aus der Basensequenz, die komplementär ist mit der Basensequenz, die in SEQ ID NO.1 dargelegt ist, hybridisiert, als ein fremdes Gen eingebaut wird, wobei die DNA, die mit der DNA, bestehend aus der Basensequenz, die komplementär ist mit der Basensequenz, die in SEQ ID NO.1 dargelegt ist, hybridisiert, für ein Polypeptid codiert, das eine differenzierungsinduzierende Promotionswirkung in einer Pankreashormon-produzierenden Zelle aufweist.

3. Verfahren zur Herstellung einer Pankreashormon-produzierenden Zelle aus einer pluripotenten Stammzelle oder einer Pankreas-Gewebestamm-/- Precursorzelle, wobei das Verfahren umfasst:
(A3) Kultivieren der Pankreas-Gewebestamm-/-Precursorzelle in Abwesenheit eines Wachstumsfaktors, der zu der TGF-β (Transformationswachstumsfaktor β) -Superfamilie, Retinoid, FGF (Fibroblastenwachstumsfaktor) und Nicotinamid gehört,
(B3) Kultivieren der in Schritt (A3) erhaltenen Zelle in Gegenwart eines Wachstumsfaktors, der zu der TGF-β-Superfamilie gehört,
(C3) Kultivieren der in Schritt (B3) erhaltenen Zelle in Gegenwart von Retinoid,
(D3) Kultivieren der in Schritt (C3) erhaltenen Zelle in Gegenwart von FGF und
(E3) Kultivieren der in Schritt (D3) erhaltenen Zelle in Gegenwart von Nicotinamid,
wobei mindestens einer der Schritte (A3) bis (E3) ein differenzierungsinduzierender Prozess von der pluripotenten Stammzelle oder der Pankreas-Gewebestamm-/-Precursorzelle zu der Pankreashormon-produzierenden Zelle ist, und
mindestens ein differenzierungsinduzierender Promotor, ausgewählt aus (1) bis (3) zu einem Kulturmedium in mindestens einem der Schritte (A3) bis (E3) gegeben wird:
(1) ein Polypeptid, bestehend aus einer Aminosäuresequenz, codiert von DNA, bestehend aus der Basensequenz, die in SEQ ID NO. 1 dargelegt ist,
(2) ein Polypeptid, bestehend aus einer Aminosäuresequenz, die eine oder mehrere Aminosäuresubstitutionen, -deletionen und / oder - additionen in der Aminosäuresequenz aufweist, die durch DNA codiert wird, bestehend aus der Basensequenz, die in SEQ ID NO. 1 dargelegt ist und eine differenzierungsinduzierende Promotionswirkung in einer Pankreashormon-produzierenden Zelle aufweist,
(3) einen Kulturüberstand von Zellen, in welche DNA, bestehend aus der Basensequenz, die in SEQ ID NO. 1 dargelegt ist oder DNA, die unter stringenten Bedingungen mit der DNA, bestehend aus der Basensequenz, die komplementär ist mit der Basensequenz, die in SEQ ID NO.1 dargelegt ist, hybridisiert, als ein fremdes Gen eingebaut wird, wobei die DNA, die mit der DNA, bestehend aus der Basensequenz, die komplementär ist mit der Basensequenz, die in SEQ ID NO.1 dargelegt ist, hybridisiert, für ein Polypeptid codiert, das eine differenzierungsinduzierende Promotionswirkung in eine Pankreashormon-produzierende Zelle aufweist.

4. Differenzierungsinduzierender Promotor zur Induzierung von Differenzierung einer pluripotenten Stammzelle oder einer Pankreas-Gewebestamm-/- Precursorzelle in eine Pankreas-Hormon-produzierende Zelle, umfassend mindestens eines von (1) bis (3):
(1) ein Polypeptid, bestehend aus einer Aminosäuresequenz, codiert durch DNA, die aus der Basensequenz besteht, die in SEQ ID NO. 1 dargelegt ist,
(2) ein Polypeptid, bestehend aus einer Aminosäuresequenz, die eine oder mehrere Aminosäuresubstitutionen, -deletionen und / oder - additionen in der Aminosäuresequenz aufweist, die durch DNA codiert wird, bestehend aus der Basensequenz, die in SEQ ID NO. 1 dargelegt ist, und eine differenzierungsinduzierende Promotionswirkung in einer Pankreas-Hormonproduzierenden Zelle aufweist,
(3) einen Kulturüberstand von Zellen, in welche DNA, bestehend aus der Basensequenz, die in SEQ ID NO. 1 dargelegt ist oder DNA, die unter stringenten Bedingungen mit der DNA, bestehend aus der Basensequenz, die komplementär ist mit der Basensequenz, die in SEQ ID NO.1 dargelegt ist, hybridisiert, als ein fremdes Gen eingebaut wird, wobei die DNA, die mit der DNA, bestehend aus der Basensequenz, die komplementär ist mit der Basensequenz, die in SEQ ID NO.1 dargelegt ist, hybridisiert, für ein Polypeptid codiert, das eine differenzierungsinduzierende Promotionswirkung in eine Pankreashormon-produzierende Zelle aufweist.

## Revendications

1. Procédé de production d'une cellule productrice d'hormone pancréatique à partir d'une cellule souche pluripotente ou d'une cellule souche/précurseur de tissu pancréatique, le procédé comprenant les étapes de :
(A1) culture de la cellule souche pluripotente en présence d'un facteur de croissance appartenant à la superfamille TGF-β (facteur de croissance transformant β),
(B1) culture de la cellule obtenue dans l'étape (A1) en présence de FGF (facteur de croissance des fibroblastes),
(C1) culture de la cellule obtenue dans l'étape (B1) en présence de rétinoïde,
(D1) culture de la cellule obtenue dans l'étape (C1) en présence d'un inhibiteur de γ-sécrétase et
(E1) culture de la cellule obtenue dans l'étape (D1) en présence d'au moins un facteur choisi dans le groupe constitué de l'exendine 4, l'HGF (facteur de croissance des hépatocytes), l'IGF-1 (facteur de croissance insulinomimétique 1) et le nicotinamide,
dans lequel au moins une des étapes (A1) à (E1) est un processus d'induction de différenciation de la cellule souche pluripotente ou la cellule souche/précurseur de tissu pancréatique en cellule productrice d'hormone pancréatique, et
au moins un promoteur d'induction de différenciation choisi parmi (1) à (3) est ajouté à un milieu de culture dans au moins une des étapes (A1) à (E1) :
(1) un polypeptide constitué d'une séquence d'acides aminés codée par un ADN constitué de la séquence de bases décrite dans SEQ ID NO. 1,
(2) un polypeptide constitué d'une séquence d'acides aminés ayant une ou plusieurs substitutions, délétions et/ou additions d'acide aminé dans la séquence d'acides aminés codée par un ADN constitué de la séquence de bases décrite dans SEQ ID NO. 1, et ayant un effet de promotion d'induction de différenciation en cellule productrice d'hormone pancréatique,
(3) un surnageant de culture de cellules dans lesquelles un ADN constitué de la séquence de bases décrite dans SEQ ID NO. 1 ou un ADN s'hybridant avec un ADN constitué d'une séquence de bases complémentaire de l'ADN constitué de la séquence de bases décrite dans SEQ ID NO: 1 dans des conditions stringentes est incorporé sous la forme d'un gène étranger, dans lequel l'ADN s'hybridant avec un ADN constitué de la séquence de bases complémentaire de l'ADN constitué de la séquence de bases décrite dans SEQ ID NO: 1 code pour un polypeptide ayant un effet de promotion d'induction de différenciation en cellule productrice d'hormone pancréatique.

2. Procédé de production d'une cellule productrice d'hormone pancréatique à partir d'une cellule souche pluripotente ou d'une cellule souche/précurseur de tissu pancréatique, le procédé comprenant les étapes de :
(A2) culture de la cellule souche pluripotente en présence d'au moins un facteur choisi dans le groupe constitué d'un facteur de croissance appartenant à la superfamille TGF-β (facteur de croissance transformant β) et un facteur de croissance appartenant à la famille Wnt (site d'intégration MMTV de type wingless) et un inhibiteur de GSK-3 (glycogène synthase kinase 3),
(B2) culture de la cellule obtenue dans l'étape (A2) en présence d'un facteur de croissance appartenant à la superfamille TGF-β,
(C2) culture de la cellule obtenue dans l'étape (B2) en présence de rétinoïde,
(D2) culture de la cellule obtenue dans l'étape (C2) en présence d'au moins un facteur choisi dans le groupe constitué d'un agent augmentant AMPc (adénosine monophosphate cyclique), la dexaméthasone, un inhibiteur d'accepteur de type TGF-β 1 et le nicotinamide,
dans lequel au moins une des étapes (A2) à (D2) est un processus d'induction de différenciation à partir de la cellule souche pluripotente ou de la cellule souche/précurseur de tissu pancréatique en cellule productrice d'hormone pancréatique, et
au moins un promoteur d'induction de différenciation choisi parmi (1) à (3) est ajouté à un milieu de culture dans au moins une des étapes (A2) à (D2) :
(1) un polypeptide constitué d'une séquence d'acides aminés codée par un ADN constitué de la séquence de bases décrite dans SEQ ID NO. 1,
(2) un polypeptide constitué d'une séquence d'acides aminés comportant une ou plusieurs substitutions, délétions et/ou additions d'acide aminé dans la séquence d'acides aminés codée par un ADN constitué de la séquence de bases décrite dans SEQ ID NO. 1, et ayant un effet de promotion d'induction de différenciation en cellule productrice d'hormone pancréatique,
(3) un surnageant de culture de cellules dans lequel un ADN constitué de la séquence de bases décrite dans SEQ ID NO. 1 ou un ADN s'hybridant avec un ADN constitué d'une séquence de bases complémentaire de l'ADN constituée de la séquence de bases décrite dans SEQ ID NO: 1 dans des conditions stringentes est incorporé sous la forme d'un gène étranger, dans lequel l'ADN s'hybridant avec un ADN constitué de la séquence de bases complémentaire de l'ADN constitué de la séquence de bases décrite dans SEQ ID NO: 1 code pour un polypeptide ayant un effet de promotion d'induction de différenciation en cellule productrice d'hormone pancréatique.

3. Procédé de production d'une cellule productrice d'hormone pancréatique à partir d'une cellule souche pluripotente ou d'une cellule souche/précurseur de tissu pancréatique, le procédé comprenant les étapes de :
(A3) culture de la cellule souche/précurseur de tissu pancréatique en l'absence d'un facteur de croissance appartenant à la superfamille TGF-β (facteur de croissance transformant β), de rétinoïde, de FGF (facteur de croissance des fibroblastes) et de nicotinamide,
(B3) culture de la cellule obtenue dans l'étape (A3) en présence d'un facteur de croissance appartenant à la superfamille TGF-β,
(C3) culture de la cellule obtenue dans l'étape (B3) en présence de rétinoïde,
(D3) culture de la cellule obtenue dans l'étape (C3) en présence de FGF et (E3) culture de la cellule obtenue dans l'étape (D3) en présence de nicotinamide,
dans lequel au moins une des étapes (A3) à (E3) est un processus d'induction de différenciation de la cellule souche pluripotente ou la cellule souche/précurseur de tissu pancréatique en cellule productrice d'hormone pancréatique, et
au moins un promoteur d'induction de différenciation choisi parmi (1) à (3) est ajouté à un milieu de culture dans au moins une des étapes (A3) à (E3) :
(1) un polypeptide constitué d'une séquence d'acides aminés codée par un ADN constitué de la séquence de bases décrite dans SEQ ID NO. 1,
(2) un polypeptide constitué d'une séquence d'acides aminés ayant une ou plusieurs substitutions, délétions et/ou additions d'acide aminé dans la séquence d'acides aminés codée par un ADN constitué de la séquence de bases décrite dans SEQ ID NO. 1, et ayant un effet de promotion d'induction de différenciation en cellule productrice d'hormone pancréatique,
(3) un surnageant de culture de cellules dans lequel un ADN constitué de la séquence de bases décrite dans SEQ ID NO. 1 ou un ADN s'hybridant avec un ADN constitué d'une séquence de bases complémentaire de l'ADN constitué de la séquence de bases décrite dans SEQ ID NO: 1 dans des conditions stringentes est incorporé sous la forme d'un gène étranger, dans lequel l'ADN s'hybridant avec un ADN constitué de la séquence de bases complémentaire de l'ADN constitué de la séquence de bases décrite dans SEQ ID NO: 1 code pour un polypeptide ayant un effet de promotion d'induction de différenciation en cellule productrice d'hormone pancréatique.

4. Utilisation d'un promoteur d'induction de différenciation pour induire la différenciation d'une cellule souche pluripotente ou d'une cellule souche/précurseur de tissu pancréatique en cellule productrice d'hormone pancréatique, comprenant au moins l'un de (1) à (3) :
(1) un polypeptide constitué d'une séquence d'acides aminés codée par un ADN constitué de la séquence de bases décrite dans SEQ ID NO. 1,
(2) un polypeptide constitué d'une séquence d'acides aminés ayant une ou plusieurs substitutions, délétions et/ou additions d'acide aminé dans la séquence d'acides aminés codée par un ADN constitué de la séquence de bases décrite dans SEQ ID NO. 1, et ayant un effet de promotion d'induction de différenciation en cellule productrice d'hormone pancréatique,
(3) un surnageant de culture de cellules dans lequel un ADN constitué de la séquence de bases décrite dans SEQ ID NO. 1 ou un ADN s'hybridant avec un ADN constitué d'une séquence de bases complémentaire de l'ADN constitué de la séquence de bases décrite dans SEQ ID NO: 1 dans des conditions stringentes est incorporé sous la forme d'un gène étranger, dans lequel l'ADN s'hybridant avec un ADN constitué de la séquence de bases complémentaire de l'ADN constitué de la séquence de bases décrite dans SEQ ID NO: 1 code pour un polypeptide ayant un effet de promotion d'induction de différenciation en cellule productrice d'hormone pancréatique.


### SEQUENCE LISTING

<110> Saitama Medical University
<120> METHOD FOR PRODUCING PANCREATIC HORMONE-PRODUCING CELL, PANCREATIC HORMONE-PRODUCING CELL, AND DIFFERENTIATION/INDUCTION PROMOTER
<130> 24-023
<150> JP 2012-120281
   <151> 2012-05-25
<160> 9
<210> 1
   <211> 2308
   <212> DNA
   <213> Homo sapiens
<400> 1
   aagccacttt gacaacgttt ctgagccagg ggtgaccatg acctgctgcg aaggatggac      60
   atcctgcaat ggattcagcc tgctggttct actgctgtta ggagtagttc tcaatgtgat     120
   acctctaatt gtcagcttag ttgaggaaga ccaattttct caaaacccca tctcttgctt     180
   tgagtggtgg ttcccaggaa ttataggagc aggtctgatg gccattccag caacaacaat     240
   gtccttgaca gcaagaaaaa gagcgtgctg caacaacaga actggaatgt ttctttcatc     300
   acttttcagt gtgatcacag tcattggtgc tctgtattgc atgctgatat ccatccaggc     360
   tctcttaaaa ggtcctctca tgtgtaattc tccaagcaac agtaatgcca attgtgaatt     420
   ttcattgaaa aacatcagtg acattcatcc agaatccttc aacttgcagt ggtttttcaa     480
   tgactcttgt gcacctccta ctggtttcaa taaacccacc agtaacgaca ccatggcgag     540
   tggctggaga gcatctagtt tccacttcga ttctgaagaa aacaaacata ggcttatcca     600
   cttctcagta tttttaggtc tattgcttgt tggaattctg gaggtcctgt ttgggctcag     660
   tcagatagtc atcggtttcc ttggctgtct gtgtggagtc tctaagcgaa gaagtcaaat     720
   tgtgtagttt aatgggaata aaatgtaagt atcagtagtt tgaattaatt tgagaagtac     780
   acttgttttc aaagtcatct ttgagatgat ttaaaaaatc aacccttcac gtagaaagca     840
   cgttgtaaat gcataacact ctcatatcag tggttgattt gggaaaggtg gagagaattt     900
   tcaattagtt ttgtgttgta ctattcaaat tttttacctc ttcactgtgt gtagagaaag     960
   gagaagggaa ggaggatgag aaggaacgga agtcatcctg aaaataaaag tacaggactt    1020
   tttttttttt tttttgagac agggtctcaa aaaaggctgg agtacagtag tacagtggtg    1080
   ctatctcagc ttactgcagc ctcaacctcc tgggctcagg tgattctccc atctcagcct    1140
   ccctagtagc tgggactaca ggtgcgtgcc actatgccaa gctaattttt gtatttttag    1200
   tagagatggg ggttttccat attgcccagg ctggtcccga actcatggac tcaagtgatc    1260
   tgcctgcctc agcctcctaa agtgctgcga ttacaggcat gagccatcgc gcctaaagga 1320
   caggaccttt ttattgtatt tctttaaaga ataaatacat aacctgaatg caatcaagtc 1380
   tttagatcta attctcagct tgcagggaac actaggacaa atccaaaaag tgggtcagcg 1440
   ggcacagaat ggcccaattt tcaacaggaa aatgttataa aagaaaaata tttttgaggg 1500
   aactgttata gattaagaga atagaggcat gtttcagcta aacacatgta aactttgtca 1560
   gagataattg ggaggagtat gtagaagaat cggattattg ttaattttgg taggtctgat 1620
   aatggtttta tagtataaag gctgagtacc ccttatccaa aatgattaag atcagaagtg 1680
   ttttggcttt cacatttttt tggattttgg aattttgcct ataataatga gacatcttgg 1740
   ggatgggatg caagtctaac cacaaaattc atttatgtct catacacact ttgaacacct 1800
   ggcctgaagg taatttcaca caatatttta aataactttg tgcatgaaac acaattttga 1860
   ctgcattttg actgcaactc atcacatgag gtcaggtatg gaattttcca cttgtggtgt 1920
   tacgttactg gctcaaaaag ttttggatct cggagcattc tggattttga atttttggat 1980
   tagtgatgct caacctgtat acagaaatgt cctcattttt aaaaaaagaa atgcatattt 2040
   atatgtttta aaattacttc aaccaaaagc aacggggaga tgtttactgt tatatttagg 2100
   tgacaggtac atggcaattc attataccct cctattttcc tatgtttaca ttattcatta 2160
   attaaaaaac aatacctaga aaaacccaag actttcaaaa gctattttct atatgtgcca 2220
   atctttaaaa aacaggataa caagggtatt tatcacatta aaatgttgta aaacagcaaa 2280
   gctaaaatct aaaaaaaaaa aaaaaaaa 2308
<210> 2
   <211> 2308
   <212> DNA
   <213> Homo sapiens
<220>
   <221> CDS
   <222> (38)..(727)
   <223>
<400> 2
   aagccacttt gacaacgttt ctgagccagg ggtgacc atg acc tgc tgc gaa gga       55
                                            Met Thr Cys Cys Glu Gly
                                            1               5
   tgg aca tcc tgc aat gga ttc agc ctg ctg gtt cta ctg ctg tta gga       103
   Trp Thr Ser Cys Asn Gly Phe Ser Leu Leu Val Leu Leu Leu Leu Gly
               10                  15                  20
   gta gtt ctc aat gtg ata cct cta att gtc agc tta gtt gag gaa gac       151
   Val Val Leu Asn Val Ile Pro Leu Ile Val Ser Leu Val Glu Glu Asp
           25                  30                  35
   caa ttt tct caa aac ccc atc tct tgc ttt gag tgg tgg ttc cca gga       199
   Gln Phe Ser Gln Asn Pro Ile Ser Cys Phe Glu Trp Trp Phe Pro Gly
       40                  45                  50
   att ata gga gca ggt ctg atg gcc att cca gca aca aca atg tcc ttg      247
   Ile Ile Gly Ala Gly Leu Met Ala Ile Pro Ala Thr Thr Met Ser Leu
   55                  60                  65                  70
   aca gca aga aaa aga gcg tgc tgc aac aac aga act gga atg ttt ctt      295
   Thr Ala Arg Lys Arg Ala Cys Cys Asn Asn Arg Thr Gly Met Phe Leu
                   75                  80                  85
   tca tca ctt ttc agt gtg atc aca gtc att ggt gct ctg tat tgc atg      343
   Ser Ser Leu Phe Ser Val Ile Thr Val Ile Gly Ala Leu Tyr Cys Met
               90                  95                  100
   ctg ata tcc atc cag gct ctc tta aaa ggt cct ctc atg tgt aat tct      391
   Leu Ile Ser Ile Gln Ala Leu Leu Lys Gly Pro Leu Met Cys Asn Ser
           105                 110                 115
   cca agc aac agt aat gcc aat tgt gaa ttt tca ttg aaa aac atc agt      439
   Pro Ser Asn Ser Asn Ala Asn Cys Glu Phe Ser Leu Lys Asn Ile Ser
       120                 125                 130
   gac att cat cca gaa tcc ttc aac ttg cag tgg ttt ttc aat gac tct      487
   Asp Ile His Pro Glu Ser Phe Asn Leu Gln Trp Phe Phe Asn Asp Ser
   135                 140                 145                 150
   tgt gca cct cct act ggt ttc aat aaa ccc acc agt aac gac acc atg      535
   Cys Ala Pro Pro Thr Gly Phe Asn Lys Pro Thr Ser Asn Asp Thr Met
                   155                 160                 165
   gcg agt ggc tgg aga gca tct agt ttc cac ttc gat tct gaa gaa aac      583
   Ala Ser Gly Trp Arg Ala Ser Ser Phe His Phe Asp Ser Glu Glu Asn
               170                 175                 180
   aaa cat agg ctt atc cac ttc tca gta ttt tta ggt cta ttg ctt gtt      631
   Lys His Arg Leu Ile His Phe Ser Val Phe Leu Gly Leu Leu Leu Val
           185                 190                 195
   gga att ctg gag gtc ctg ttt ggg ctc agt cag ata gtc atc ggt ttc      679
   Gly Ile Leu Glu Val Leu Phe Gly Leu Ser Gln Ile Val Ile Gly Phe
       200                 205                 210
   ctt ggc tgt ctg tgt gga gtc tct aag cga aga agt caa att gtg tag      727
   Leu Gly Cys Leu Cys Gly Val Ser Lys Arg Arg Ser Gln Ile Val
   215                 220                 225
   tttaatggga ataaaatgta agtatcagta gtttgaatta atttgagaag tacacttgtt    787
   ttcaaagtca tctttgagat gatttaaaaa atcaaccctt cacgtagaaa gcacgttgta    847
   aatgcataac actctcatat cagtggttga tttgggaaag gtggagagaa ttttcaatta    907
   gttttgtgtt gtactattca aattttttac ctcttcactg tgtgtagaga aaggagaagg    967
   gaaggaggat gagaaggaac ggaagtcatc ctgaaaataa aagtacagga cttttttttt   1027
   tttttttgga gacagggtct caaaaaaggc tggagtacag tagtacagtg gtgctatctc   1087
   agcttactgc agcctcaacc tcctgggctc aggtgattct cccatctcag cctccctagt   1147
   agctgggact acaggtgcgt gccactatgc caagctaatt tttgtatttt tagtagagat   1207
   gggggttttc catattgccc aggctggtcc cgaactcatg gactcaagtg atctgcctgc   1267
   ctcagcctcc taaagtgctg cgattacagg catgagccat cgcgcctaaa ggacaggacc   1327
   tttttattgt atttctttaa agaataaata cataacctga atgcaatcaa gtctttagat   1387
   ctaattctca gcttgcaggg aacactagga caaatccaaa aagtgggtca gcgggcacag   1447
   aatggcccaa ttttcaacag gaaaatgtta taaaagaaaa atatttttga gggaactgtt   1507
   atagattaag agaatagagg catgtttcag ctaaacacat gtaaactttg tcagagataa   1567
   ttgggaggag tatgtagaag aatcggatta ttgttaattt tggtaggtct gataatggtt   1627
   ttatagtata aaggctgagt accccttatc caaaatgatt aagatcagaa gtgttttggc   1687
   tttcacattt ttttggattt tggaattttg cctataataa tgagacatct tggggatggg   1747
   atgcaagtct aaccacaaaa ttcatttatg tctcatacac actttgaaca cctggcctga   1807
   aggtaatttc acacaatatt ttaaataact ttgtgcatga aacacaattt tgactgcatt   1867
   ttgactgcaa ctcatcacat gaggtcaggt atggaatttt ccacttgtgg tgttacgtta   1927
   ctggctcaaa aagttttgga tctcggagca ttctggattt tgaatttttg gattagtgat   1987
   gctcaacctg tatacagaaa tgtcctcatt tttaaaaaaa gaaatgcata tttatatgtt   2047
   ttaaaattac ttcaaccaaa agcaacgggg agatgtttac tgttatattt aggtgacagg   2107
   tacatggcaa ttcattatac cctcctattt tcctatgttt acattattca ttaattaaaa   2167
   aacaatacct agaaaaaccc aagactttca aaagctattt tctatatgtg ccaatcttta   2227
   aaaaacagga taacaagggt atttatcaca ttaaaatgtt gtaaaacagc aaagctaaaa   2287
   tctaaaaaaa aaaaaaaaaa a                                              2308
<210> 3
   <211> 229
   <212> PRT
   <213> Homo sapiens
<400> 3
   Met Thr Cys Cys Glu Gly Trp Thr Ser Cys Asn Gly Phe Ser Leu Leu
   1               5                   10                  15
   Val Leu Leu Leu Leu Gly Val Val Leu Asn Val Ile Pro Leu Ile Val
               20                  25                  30
   Ser Leu Val Glu Glu Asp Gln Phe Ser Gln Asn Pro Ile Ser Cys Phe
           35                  40                  45
   Glu Trp Trp Phe Pro Gly Ile Ile Gly Ala Gly Leu Met Ala Ile Pro
       50                  55                  60
   Ala Thr Thr Met Ser Leu Thr Ala Arg Lys Arg Ala Cys Cys Asn Asn
   65                  70                  75                  80
   Arg Thr Gly Met Phe Leu Ser Ser Leu Phe Ser Val Ile Thr Val Ile
                   85                  90                  95
   Gly Ala Leu Tyr Cys Met Leu Ile Ser Ile Gln Ala Leu Leu Lys Gly
               100                 105                 110
   Pro Leu Met Cys Asn Ser Pro Ser Asn Ser Asn Ala Asn Cys Glu Phe
           115                 120                 125
   Ser Leu Lys Asn Ile Ser Asp Ile His Pro Glu Ser Phe Asn Leu Gln
       130                 135                 140
   Trp Phe Phe Asn Asp Ser Cys Ala Pro Pro Thr Gly Phe Asn Lys Pro
   145                 150                 155                 160
   Thr Ser Asn Asp Thr Met Ala Ser Gly Trp Arg Ala Ser Ser Phe His
                   165                 170                 175
   Phe Asp Ser Glu Glu Asn Lys His Arg Leu Ile His Phe Ser Val Phe
               180                 185                 190
   Leu Gly Leu Leu Leu Val Gly Ile Leu Glu Val Leu Phe Gly Leu Ser
           195                 200                 205
   Gln Ile Val Ile Gly Phe Leu Gly Cys Leu Cys Gly Val Ser Lys Arg
       210                 215                 220
   Arg Ser Gln Ile Val
   225
<210> 4
   <211> 20
   <212> DNA
   <213> Artificial sequence
<220>
   <223> Synthesized oligonucleotide
<400> 4
   gcatttactt tgtggctgga 20
<210> 5
   <211> 20
   <212> DNA
   <213> Artificial sequence
<220>
   <223> Synthesized oligonucleotide
<400> 5
   cctgggaagc tgagaatgat 20
<210> 6
   <211> 20
   <212> DNA
   <213> Artificial sequence
<220>
   <223> Synthesized oligonucleotide
<400> 6
   ccccagactc cgtcagtttc 20
<210> 7
   <211> 19
   <212> DNA
   <213> Artificial sequence
<220>
   <223> Synthesized oligonucleotide
<400> 7
   tccgtctggt tgggttcag 19
<210> 8
   <211> 19
   <212> DNA
   <213> Artificial sequence
<220>
   <223> Synthesized oligonucleotide
<400> 8
   cacttcctac ccctgctgg 19
<210> 9
   <211> 19
   <212> DNA
   <213> Artificial sequence
<220>
   <223> Synthesized oligonucleotide
<400> 9
   acgccaaggt ctgaaggtc 19